# EUROPEAN PATENT SPECIFICATION

(11) **EP 1 673 086 B1**
(45) Date of publication and mention of the grant of the patent: **23.01.2008**
(21) Application number: 04765656.6
(22) Date of filing: 23.09.2004
(51) Int. Cl.: A61K 31/4706, C07D 215/54, C07D 405/12, C07D 401/12, C07D 401/14, C07D 405/14, A61K 31/4709, A61P 29/00, A61P 11/00

(54) **4-AMINOQUINOLINE-3-CARBOXAMIDE DERIVATIVES AS PDE4 INHIBITORS**
4-AMINOCHINOLIN-3-CARBONSÄUREAMID-DERIVATE ALS PDE4-HEMMER
DERIVES DE 4-AMINOQUINOLINE-3-CARBOXAMIDE UTILISES COMME INHIBITEURS DE PDE4

(30) Priority: 27.09.2003 GB 0322722
(43) Date of publication of application: 28.06.2006
(73) Proprietor: GLAXO GROUP LIMITED, Greenford, Middlesex UB6 0NN (GB)
(72) Inventor: EDLIN, Christopher, D. GlaxoSmithKline, Stevenage, Hertfordshire SG1 2NY (GB); ELDRED, Colin, David GlaxoSmithKline, Stevenage, Hertfordshire SG1 2NY (GB); KEELING, Steven, Philip GlaxoSmithKline, Stevenage, Hertfordshire SG1 2NY (GB); LUNNISS, Christopher, James GlaxoSmithKline, Stevenage, Hertfordshire SG1 2NY (GB); REDFERN, Tracy, Jane GlaxoSmithKline, Stevenage, Hertfordshire SG1 2NY (GB); REDGRAVE, Alison, Judith GlaxoSmithKline, Stevenage, Hertfordshire SG1 2NY (GB); WOODROW, Michael GlaxoSmithKline, Stevenage, Hertfordshire SG1 2NY (GB)
(74) Representative: Learoyd, Stephanie Anne
(86) International application number: PCT/EP2004/010844
(87) International publication number: WO 2005/030212

(56) References cited:
- WO-A-02/20489
- WO-A-97/44322
- WO-A-02/092571

## Description

The present invention relates to quinoline compounds, processes for their preparation, intermediates usable in these processes, and pharmaceutical compositions containing the compounds. The invention also relates to the use of the quinoline compounds in therapy, for example as inhibitors of phosphodiesterases and/or for the treatment and/or prophylaxis of inflammatory and/or allergic diseases such as chronic obstructive pulmonary disease (COPD), asthma, rheumatoid arthritis or allergic rhinitis.

WO 02/20489 A2 (Bristol-Myers-Squibb Company) discloses 4-aminoquinoline derivatives wherein the 4-amino group NR⁴R⁵ may represent an acyclic amino group wherein R⁴ and R⁵ may each independently represent hydrogen, alkyl, cycloalkyl, aryl, heteroaryl etc.; NR⁴R⁵ may alternatively represent an aliphatic heterocyclic group. The compounds are disclosed as inhibitors of cGMP phosphodiesterase, especially type 5 (PDE5).

EP 0 480 052 (Otsuka Pharmaceutical Co. Ltd.) discloses 4-aminoquinoline-3-carboxamides wherein the 4-amino group NHR⁴ may represent an amino group wherein R⁴ represents phenyl, tetrahydronaphthyl or naphthyl, optionally substituted with alkyl, halogen, alkoxy etc.; and the 3-carboxamide group CONR²R³ represents a primary, secondary or tertiary carboxamide group. The compounds are disclosed as inhibitors of gastric acid secretion, and as cytoprotective agents; inhibition of the ATPase activated by H⁺ and K⁺ at the gastric wall cells is also disclosed.

It is desirable to find new compounds which bind to, and preferably inhibit, phosphodiesterase type IV (PDE4).

According to the invention there is provided a compound of formula (I) or a pharmaceutically acceptable salt thereof: 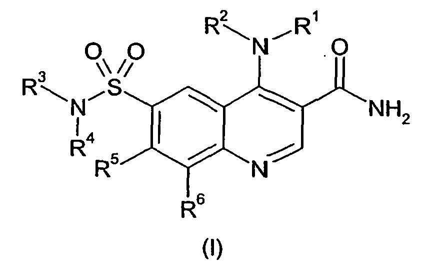 wherein:
R¹ is
   Aryl optionally substituted by one or more substituents selected from C₁₋₆alkyl, C₁₋₆alkoxy, halogen, C₁₋₆alkylCO-, -(CH₂)ₘOH, -CN, R⁷R⁸N-;
   Aryl fused to a C₄₋₇cycloalkyl ring;
   Aryl fused to a heterocyclyl ring;
   Heteroaryl wherein the heteroaryl is optionally substituted by one or more substituents selected from: C₁₋₆alkyl, N-oxide, C₁₋₆alkoxy;
   Heterocyclyl.
R² is hydrogen or C₁₋₆alkyl;
R³ is
   Hydrogen;
   C₁₋₆alkyl optionally substituted by one or more substituents selected from: heterocyclyl (itself optionally substituted by C₁₋₆alkyl), R⁹R¹⁰NCO-, R¹¹CONR¹²-, C₁₋₆alkylSO₂NR¹³-, C₁₋₆alkoxy, R¹⁴R¹⁵N-;
   C₃₋₇cycloalkyl;
   Aryl or aryl(C₁₋₆alkyl) wherein the aryl is optionally substituted by one or more substituents selected from: C₁₋₆alkyl, C₁₋₆alkoxy, halogen, R¹⁶R¹⁷NCO-;
   Aryl fused to C₄₋₇cycloalkyl, wherein the cycloalkyl is optionally substituted by =O;
   Heteroaryl or heteroaryl(C₁₋₆alkyl), wherein the heteroaryl is optionally substituted by one or more substituents selected from C₁₋₆alkyl, C₁₋₆alkoxy, halogen;
   Heterocyclyl optionally substituted by one or more C₁₋₆alkyl, C₁₋₆alkylCO-, C₁₋₆alkylSO₂-, R¹⁸R¹⁹NCO-, C₁₋₆alkoxyCO-;
R⁴ is hydrogen or C₁₋₆alkyl;
R³ and R⁴ together with the nitrogen atom to which they are attached may form a heterocyclyl ring, which is optionally substituted by one or more substituents selected from C₁₋₆alkyl (optionally substituted by one or more OH or C₁₋₆alkoxy groups), C₁₋₆alkoxy, C_{1- 6}alkoxyCO-, C₃₋₇cycloalkyl (optionally substituted by OH), C₁₋₆alkylCO-, C₁₋₆alkylSO₂-, OH, -(CH₂)ₘNR²⁰R²¹, -(CH₂)ₘCONR²²R²³, -(CH₂)ₘNR²⁴COR²⁵, C₁₋₆alkoxyC₁₋₄alkyl, arylCO-heteroaryl, heteroarylC₁₋₄alkyl, heteroarylCO.
m is 0-6
R⁵ is hydrogen or C₁₋₆alkyl;
R⁶ is hydrogen, C₁₋₆alkyl, C₁₋₆alkoxy, fluorine, chlorine, or bromine;
R⁷⁻²⁵ all independently represent hydrogen, C₁₋₆ alkyl;
R¹⁴ and R¹⁵ together with the nitrogen atom to which they are attached may form a heterocyclyl ring;
R¹⁶ and R¹⁷ together with the nitrogen atom to which they are attached may form a heterocyclyl ring;
R¹⁸ and R¹⁹ together with the nitrogen atom to which they are attached may form a heterocyclyl ring;
R²⁰ and R²¹ together with the nitrogen atom to which they are attached may form a heterocyclyl ring;
R²² and R²³ together with the nitrogen atom to which they are attached may form a heterocyclyl ring;

As used herein, the term "alkyl" refers to straight or branched hydrocarbon chains containing the specified number of carbon atoms. For example, C₁₋₆alkyl means a straight or branched alkyl chain containing at least 1, and at most 6, carbon atoms. Examples of "alkyl" as used herein include, but are not limited to, methyl, ethyl, n-propyl, iso-propyl, n-butyl, sec-butyl, iso-butyl, *t*-butyl, n-pentyl and *n*-hexyl. A C₁₋₄alkyl group is preferred, for example methyl, ethyl or isopropyl. The said alkyl groups may be optionally substituted with one or more fluorine atoms, for example, trifluoromethyl.

As used herein, the term "alkoxy" refers to a straight or branched chain alkoxy group, for example, methoxy, ethoxy, prop-1-oxy, prop-2-oxy, but-1-oxy, but-2-oxy, 2-methylprop-1-oxy, 2-methylprop-2-oxy, pentoxy or hexyloxy. A C₁₋₄alkoxy group is preferred, for example methoxy or ethoxy. The said alkoxy groups may be optionally substituted with one or more fluorine atoms, for example, trifluoromethoxy.

As used herein, the term "cycloalkyl" refers to a non-aromatic hydrocarbon ring containing the specified number of carbon atoms. For example, C₃₋₇cycloalkyl means a non-aromatic ring containing at least three, and at most seven, ring carbon atoms. Examples of "cycloalkyl" as used herein include, but are not limited to, cyclopropyl, cyclobutyl, cyclopentyl, cyclohexyl and cycloheptyl. A C₃₋₆cycloalkyl group is preferred, for example cyclopentyl.

When used herein, the term "aryl" refers to, unless otherwise defined, a mono- or bicyclic carbocyclic aromatic ring system containing up to 10 carbon atoms in the ring system, for instance phenyl or naphthyl, optionally fused to a C₄₋₇cycloalkyl or heterocyclyl ring.

As used herein, the terms "heteroaryl ring" and "heteroaryl" refer to a monocyclic five- to seven- membered heterocyclic aromatic ring containing one or more heteroatoms selected from oxygen, nitrogen and sulfur. In a particular aspect such a ring contains 1-3 heteroatoms. Preferably, the heteroaryl ring has five or six ring atoms. Examples of heteroaryl rings include, but are not limited to, furyl, thienyl, pyrrolyl, oxazolyl, thiazolyl, isoxazolyl, isothiazolyl, imidazolyl, pyrazolyl, oxadiazolyl, triazolyl, tetrazolyl, thiadiazolyl, pyridinyl, pyridazinyl, pyrimidinyl, pyrazinyl and triazinyl. The terms "heteroaryl ring" and "heteroaryl" also refer to fused bicyclic heterocyclic aromatic ring systems containing at least one heteroatom selected from oxygen, nitrogen and sulfur. Preferably, the fused rings each have five or six ring atoms. Examples of fused heterocyclic aromatic rings include, but are not limited to, quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl and benzothiadiazolyl. The heteroaryl may attach to the rest of the molecule through any atom with a free valence.

As used herein, the term "heterocyclyl" refers to a monocyclic three- to seven-membered saturated or non-aromatic, unsaturated ring containing at least one heteroatom selected from oxygen, nitrogen and sulfur. In a particular aspect such a ring contains 1 or 2 heteroatoms. Preferably, the heterocyclyl ring has five or six ring atoms. Examples of heterocyclyl groups include, but are not limited to, aziridinyl, azetidinyl, pyrrolidinyl, piperidinyl, imidazolidinyl, pyrazolidinyl, piperazinyl, morpholinyl, thiomorpholinyl, diazepinyl, azepinyl, tetrahydrofuranyl, tetrahydropyranyl, and 1,4-dioxanyl.

As used herein, the terms "halogen" or "halo" refer to fluorine, chlorine, bromine and iodine. Preferred halogens are fluorine, chlorine and bromine. Particularly preferred halogens are fluorine and chlorine.

As used herein, the term "optionally" means that the subsequently described event(s) may or may not occur, and includes both event(s) which occur and events that do not occur.

As used herein, the term "substituted" refers to substitution with the named substituent or substituents, multiple degrees of substitution being allowed unless otherwise stated.

In a preferred embodiment, R¹ is selected from
Aryl optionally substituted by one or more substituents selected from C₁₋₆alkyl, C₁₋₆alkoxy-, halogen, -CN;
Aryl fused to a heterocyclyl ring;
Heteroaryl optionally substituted by one or more substituents selected from: C₁₋₆alkyl.

In a preferred embodiment R¹ is selected from
Phenyl optionally substituted by one or more substituents selected from methyl, methoxy, fluoro, chloro, cyano;
Dihydrobenzofuranyl ;
Indazolyl or benzimidazolyl optionally substituted by methyl.

Representative examples of R¹ include 3-(methyloxy)phenyl, 3-chlorophenyl, 3-fluorophenyl, 3-cyanophenyl, 3-methylphenyl, 4-fluoro-3-(methyloxy)phenyl, 1-methyl-1H-benzimidazol-6-yl, 1-methyl-1 H-indazol-6-yl and 2,3-dihydro-1-benzofuran-4-yl.

In a preferred embodiment, R² is hydrogen.

Representative examples of R² include hydrogen.

In a preferred embodiment R³ is selected from
C₁₋₆alkyl optionally substituted by one or more C₁₋₆alkoxy groups;
C₃₋₇cycloalkyl;
Heterocyclyl.

In a preferred embodiment R³ is selected from
C₁₋₆alkyl optionally substituted by one or more substituents selected from heterocyclyl, C₁₋₆alkoxy;
C₃₋₇cycloalkyl;
Heterocyclyl.

In a preferred embodiment R³ is selected from
C₁₋₃alkyl optionally substituted by one C₁₋₂alkoxy group or a 5 to 7 membered saturated ring containing one or two heteratoms selected from nitrogen or oxygen;
C₃₋₅cycloalkyl;
5 to 7 membered saturated ring containing one heteroatom which is oxygen.

Representative examples of R³ include methyl, 2-(methyloxy)ethyl, tetrahydro-2H-pyran-4-yl, cyclopropyl, N-pyrrolidinethyl and N-morpholinethyl.

In a preferred embodiment R⁴ is hydrogen or C₁₋₆alkyl;

Representative examples of R⁴ include hydrogen and methyl.

In a particularly preferred embodiment, R³and R⁴ together with the nitrogen atom to which they are attached may form a heterocyclyl ring, optionally substituted by C₁₋₆alkyl (optionally substituted by one or more C₁₋₆alkoxy groups), C₁₋₆alkylCO, C₁₋₆alkyISO₂; - (CH₂)ₘCONR₂₂R²³, heteroaryl.

In a particularly preferred embodiment, R³ and R⁴ together with the nitrogen atom to which they are attached may form a heterocyclyl ring, optionally substituted by one or more substituents selected from C₁₋₆alkyl (optionally substituted by one or more C₁₋₆alkoxy groups), C₁₋₆alkylCO, C₁₋₆alkylSO₂; -(CH₂)ₘCONR²²R²³, -(CH₂)ₘNR²⁰R²¹, heteroaryl.

In a particularly preferred embodiment, R³ and R⁴ together with the nitrogen atom to which they are attached may form a 5 or 6 membered heterocyclyl ring, optionally substituted by one or more substituents selected from C₁₋₃alkyl (optionally substituted by one or more C₁₋₂alkoxy groups), C₁₋₃alkylCO, C₁₋₃alkylSO₂; -CON(CH₃)₂, -N(CH₃)₂, pyrazinyl, pyridinyl.

Representative examples wherein R³ and R⁴ together with the nitrogen atom to which they are attached may form a heterocyclyl ring include 1-piperidinyl, 4-morpholinyl, 4-methyl-1-piperazinyl, 4-acetyl-1-piperazinyl, 4-(methylsulfonyl)-1-piperazinyl, 4-(2-pyrazinyl)-1-piperazinyl, 4-[2-(methyloxy)ethyl]-1-piperazinyl, 4-[(dimethylamino)carbonyl]-1-piperazinyl, 1-pyrrolidinyl and 4- (dimethylamino)-1-piperidinyl.

In a preferred embodiment R⁵ represents hydrogen.

Representative examples of R⁵ include hydrogen.

In a preferred embodiment R⁶ represents hydrogen or C₁₋₆alkyl.

Representative examples of R⁶ include hydrogen and methyl.

In a preferred embodiment there exists a subgroup of formula (IA) wherein
R¹ is selected from
   phenyl optionally substituted by one or more substituents selected from methyl, methoxy, fluoro, chloro, cyano;
   dihydrobenzofuranyl;
   indazolyl or benzimidazolyl optionally substituted by methyl;
R² is hydrogen;
R³ is selected from
   C₁₋₃alkyl optionally substituted by one C₁₋₂alkoxy group or a 5 to 7 membered saturated ring containing one or two heteratoms selected from nitrogen or oxygen;
   C₃₋₅cycloalkyl;
   5 to 7 membered saturated ring containing one heteroatom which is oxygen;
R⁴ is hydrogen or C₁₋₆alkyl;
R⁵ is hydrogen;
R⁶ is hydrogen or C₁₋₆alkyl.

In a preferred embodiment there exists a subgroup of formula (IB) wherein
R¹ is selected from
   phenyl optionally substituted by one or more substituents selected from methyl, methoxy, fluoro, chloro, cyano;
   dihydrobenzofuranyl;
   indazolyl or benzimidazolyl optionally substituted by methyl;
R² is hydrogen;
R³ and R⁴ together with the nitrogen atom to which they are attached may form a 5 or 6 membered heterocyclyl ring, optionally substituted by one or more substituents selected from C₁₋₃alkyl (optionally substituted by one or more C₁₋₂alkoxy groups), C₁₋₃alkylCO, C₁₋₃alkylSO₂; -CON(CH₃)₂, -N(CH₃)₂, pyrazinyl, pyridinyl;
R⁵ is hydrogen;
R⁶ is hydrogen or C₁₋₆alkyl.

It is to be understood that the present invention covers all combinations of substituent groups referred to hereinabove.

It is to be understood that the present invention covers all combinations of particular and preferred groups described hereinabove.

Particular compounds according to the invention include those mentioned in the examples and their pharmaceutically acceptable salts. Specific examples which may be mentioned include:
Example 2: 6-[(dimethylamino)sulfonyl]-4-{[3-(methyloxy)phenyl]amino}-3-quinolinecarboxamide
Example 5: 4-(2,3-dihydro-1-benzofuran-4-ylamino)-6-(4-morpholinylsulfonyl)-3-quinolinecarboxamide
Example 11: 6-[(4-acetyl-1-piperazinyl)sulfonyl]-4-{[4-fluoro-3-(methyloxy)phenyl]amino}-3-quinolinecarboxamide
Example 12: 4-{[4-fluoro-3-(methyloxy)phenyl]amino}-6-{[4-(methylsulfonyl)-1-piperazinyl]sulfonyl}-3-quinolinecarboxamide
Example 13: 6-[(4-acetyl-1-piperazinyl)sulfonyl]-4-(2,3-dihydro-1-benzofuran-4-ylamino)-3-quinolinecarboxamide
Example 14: 4-(2,3-dihydro-1-benzofuran-4-ylamino)-6-{[4-(methylsulfonyl)-1-piperazinyl]sulfonyl}-3-quinolinecarboxamide
Example 17: 4-(2,3-dihydro-1-benzofuran-4-ylamino)-6-[(dimethylamino)sulfonyl]-3-quinolinecarboxamide
Example 21: 6-({4-[(dimethylamino)carbonyl]-1-piperazinyl}sulfonyl)-4-{[4-fluoro-3-(methyloxy)phenyl]amino}-3-quinolinecarboxamide
Example 22: 4-(2,3-dihydro-1-benzofuran-4-ylamino)-6-{[4-(2-pyrazinyl)-1-piperazinyl]sulfonyl}-3-quinolinecarboxamide
Example 23: 4-(2,3-dihydro-1-benzofuran-4-ylamino)-6-({4-[(dimethylamino)carbonyl]-1-piperazinyl}sulfonyl)-3-quinolinecarboxamide
Example 29:4-(2,3-dihydro-1-benzofuran-4-ylamino)-6-[(tetrahydro-2*H*-pyran-4-ylamino)sulfonyl]-3-quinolinecarboxamide
and pharmaceutically acceptable salts thereof.

Further specific examples which may be mentioned include:
Example 42: 4-{[4-fluoro-3-(methyloxy)phenyl]amino}-8-methyl-6-(4-morpholinylsulfonyl)-3-quinolinecarboxamide
Example 43: 4-(2,3-dihydro-1-benzofuran-4-ylamino)-8-methyl-6-(4-morpholinylsulfonyl)-3-quinolinecarboxamide
Eample 44: 8-methyl-4-[(3-methylphenyl)amino]-6-(4-morpholinylsulfonyl)-3-quinolinecarboxamide
Example 45: 4-[(3-fluorophenyl)amino]-8-methyl-6-(4-morpholinylsulfonyl)-3-quinolinecarboxamide
Example 46: 4-[(3-cyanophenyl)amino]-8-methyl-6-(4-morpholinylsulfonyl)-3-quinolinecarboxamide
Example 48: 4-(2,3-dihydro-1-benzofuran-4-ylamino)-6-{[4-(dimethylamino)-1-piperidinyl]sulfonyl}-3-quinolinecarboxamide
Example 50: 4-[(3-chlorophenyl)amino]-8-methyl-6-(4-morpholinylsulfonyl)-3-quinolinecarboxamide
Example 51: 8-methyl-4-[(1-methyl-1H-indazol-6-yl)amino]-6-(4-morpholinylsulfonyl)-3-quinolinecarboxamide
Example 52: 6-[(4-acetyl-1-piperazinyl)sulfonyl]-8-methyl-4-[(3-methylphenyl)amino]-3-quinolinecarboxamide
Example 53: 6-[(4-acetyl-1-piperazinyl)sulfonyl]-4-{[4-fluoro-3-(methyloxy)phenyl]amino}-8-methyl-3-quinolinecarboxamide
Example 54: 6-[(4-acetyl-1-piperazinyl)sulfonyl]-4-(2,3-dihydro-1-benzofuran-4-ylamino)-8-methyl-3-quinolinecarboxamide
and pharmaceutically acceptable salts thereof.

Preferred specific examples which may be mentioned include:
Example 23: 4-(2,3-dihydro-1-benzofuran-4-ylamino)-6-({4-[(dimethylamino)carbonyl]-1-piperazinyl}sulfonyl)-3-quinolinecarboxamide
Example 51: 8-methyl-4-[(1-methyl-1H-indazol-6-yl)amino]-6-(4-morpholinylsulfonyl)-3-quinolinecarboxamide
Example 54: 6-[(4-acetyl-1-piperazinyl)sulfonyl]-4-(2,3-dihydro-1-benzofuran-4-ylamino)-8-methyl-3-quinolinecarboxamide
and pharmaceutically acceptable salts thereof.

Further preferred specific examples which may be mentioned include:
Example 11: 6-[(4-acetyl-1-piperazinyl)sulfonyl]-4-{[4-fluoro-3-(methyloxy)phenyl]amino}-3-quinolinecarboxamide
Example 13: 6-[(4-acetyl-1-piperazinyl)sulfonyl]-4-(2,3-dihydro-1-benzofuran-4-ylamino)-3-quinolinecarboxamide
Example 50: 4-[(3-chlorophenyl)amino]-8-methyl-6-(4-morpholinylsulfonyl)-3-quinolinecarboxamide
Example 52: 6-[(4-acetyl-1-piperazinyl)sulfonyl]-8-methyl-4-[(3-methylphenyl)amino]-3-quinolinecarboxamide
Example 53: 6-[(4-acetyl-1-piperazinyl)sulfonyl]-4-{[4-fluoro-3-(methyloxy)phenyl]amino}-8-methyl-3-quinolinecarboxamide
and pharmaceutically acceptable salts thereof.

Salts of the compounds of the present invention are also encompassed within the scope of the invention. Because of their potential use in medicine, the salts of the compounds of formula (I) are preferably pharmaceutically acceptable. Suitable pharmaceutically acceptable salts can include acid or base addition salts. A. pharmaceutically acceptable acid addition salt can be formed by reaction of a compound of formula (I) with a suitable inorganic or organic acid (such as hydrobromic, hydrochloric, sulfuric, nitric, phosphoric, succinic, maleic, acetic, fumaric, citric, tartaric, benzoic, p-toluenesulfonic, methanesulfonic or naphthalenesulfonic acid), optionally in a suitable solvent such as an organic solvent, to give the salt which is usually isolated for example by crystallisation and filtration. A pharmaceutically acceptable acid addition salt of a compound of formula (I) can be for example a hydrobromide, hydrochloride, sulfate, nitrate, phosphate, succinate, maleate, acetate, fumarate, citrate, tartrate, benzoate, p-toluenesulfonate, methanesulfonate or naphthalenesulfonate salt. A pharmaceutically acceptable base addition salt can be formed by reaction of a compound of formula (I) with a suitable inorganic or organic base, optionally in a suitable solvent such as an organic solvent, to give the base addition salt which is usually isolated for example by crystallisation and filtration. Other non-pharmaceutically acceptable salts, eg. oxalates or trifluoroacetates, may be used, for example in the isolation of compounds of the invention, and are included within the scope of this invention. The invention includes within its scope all possible stoichiometric and non-stoichiometric forms of the salts of the compounds of formula (I). Also included within the scope of the invention are all solvates, hydrates and complexes of compounds and salts of the invention.

Certain compounds of formula (I) may exist in stereoisomeric forms (e.g. they may contain one or more asymmetric carbon atoms or may exhibit *cis-trans* isomerism). The individual stereoisomers (enantiomers and diastereomers) and mixtures of these are included within the scope of the present invention. The present invention also covers the individual isomers of the compounds represented by formula (I) as mixtures with isomers thereof in which one or more chiral centres are inverted. Likewise, it is understood that compounds of formula (I) may exist in tautomeric forms other than that shown in the formula and these are also included within the scope of the present invention.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the working Examples.

### Process a

Compounds of formula (I), wherein R¹, R², R³, R⁴, R⁵ and R⁶ are as defined above may be prepared from compounds of formula (II); 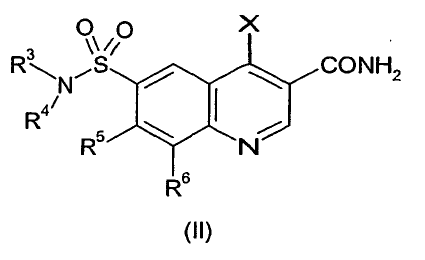 wherein R³, R⁴, R⁵ and R⁶ are as defined above, and X represents a halogen atom, by treatment with an amine of formula R¹R²NH, wherein R¹ and R² are as defined above.

Suitable conditions for process a) include stirring in a suitable solvent such as acetonitrile or a mixture of acetonitrile and *N,N*-dimethylformamide, at a suitable temperature, such as between room temperature and the reflux temperature of the solvent, for example at 80°C, optionally in the presence of a suitable base such a *N,N*-diisopropylethylamine.

Alternatively, process a) may be carried out under microwave irradiation, at a suitable power such as 150W, in a suitable solvent such as *N*-methyl-2-pyrrolidinone, at a suitable temperature such as 60-200°C, for example at 150°C. Process a) may alternatively be carried out in the presence of an acid catalyst, such as pyridine hydrochloride or concentrated hydrochloric acid, in a suitable solvent such as ethanol, at a suitable temperature such as the reflux temperature of the solvent.

Compounds of formula (II), wherein R³, R⁴, R⁵, R⁶, and X are as defined above, may be prepared from compounds of formula (III); 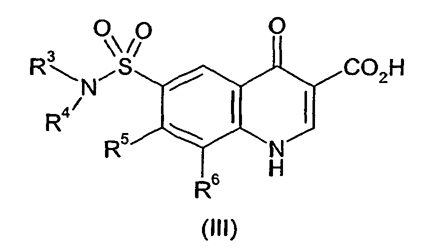 wherein R³, R⁴, R⁵ and R⁶ are as defined above, by treatment with a suitable halogenating agent, such as a chlorinating agent, for example thionyl chloride, in the presence of a suitable catalyst such as *N,N*-dimethylformamide, followed by treatment with ammonia under suitable conditions, such as 880 ammonia at room temperature.

Compounds of formula (III), wherein R³, R⁴, R⁵ and R⁶are as defined above, may be prepared from compounds of formula (IV); 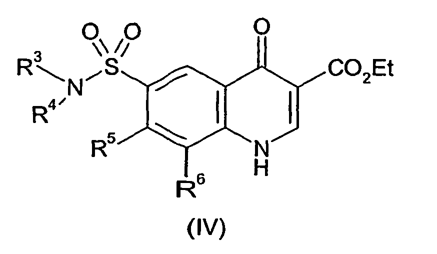 wherein R³, R⁴, R⁵ and R⁶ are as defined above, by hydrolysis with a suitable base, such as aqueous sodium hydroxide, in a suitable solvent, such as ethanol, at a suitable temperature such as room temperature.

Compounds of formula (IV), wherein R³, R⁴, R⁵ and R⁶ are as defined above, may be prepared from compounds of formula (V); 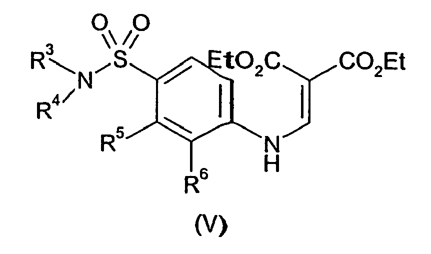 wherein R³, R⁴, R⁵ and R⁶ are as defined above, by heating in a suitable solvent, such as diphenyl ether, at a suitable temperature such as 200-300°C, for example at 250°C.

Compounds of formula (V), wherein R³, R⁴, R⁵ and R⁶ are as defined above, may be prepared from compounds of formula (VI), wherein R³, R⁴, R⁵ and R⁶ are as defined above, and the compound of formula (VII);

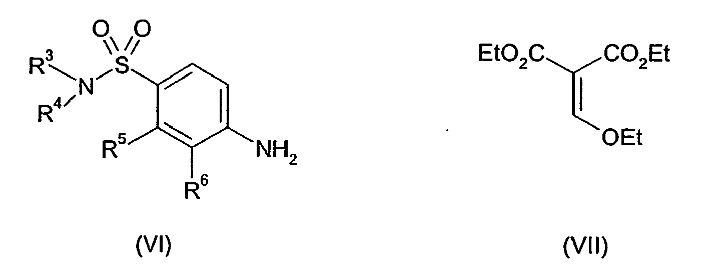

Suitable conditions include heating together compounds of formulae (VI) and (VII) in the absence of solvent, at a suitable temperature, such as 60-150°C, for example at 100°C. Alternative conditions include heating together compounds of formulae (VI) and (VII) under microwave irradiation, for example at 150W power, at a suitable temperature such as 100-200°C, for example at 150°C, for a suitable time such as 15 minutes.

Preparation of compounds of formula (III), (IV) and (V) wherein R³R⁴N represents dimethylamino, diethylamino, or di(*n*-propylamino), R⁵ = H and R⁶ = H; or wherein R³ and R⁴ together with the nitrogen to which they are attached represent 1-piperidinyl, 4-morpholinyl and 4-methyl-1-piperazinyl, R⁵ = H and R⁶ = H have been previously described in patent ZA 6706075 (1968).

The compounds of formula (VI) wherein R⁵ and R⁶ both represent hydrogen and R³ and R⁴ both represent methyl (SALOR); or wherein R⁵ and R⁶ both represent hydrogen and R³ and R⁴ together with the nitrogen to which they are attached represent morpholine (Maybridge Int) or piperidine (Maybridge Int) are commercially available.

The compound of formula (VII) is commercially available (Aldrich).

Compounds of formula (VI), wherein R³, R⁴, R⁵ and R⁶ are as defined above, may be prepared from compounds of formula (VIII); 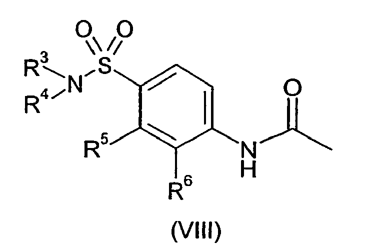 wherein R³, R⁴, R⁵ and R⁶ are as defined above, by hydrolysis with a suitable base, such as aqueous sodium hydroxide, in a suitable solvent, such as ethanol, at a suitable temperature such as 80°C.

Compounds of formula (VIII), wherein R³, R⁴, R⁵ and R⁶ are as defined above, may be prepared from compounds of formula (IX); 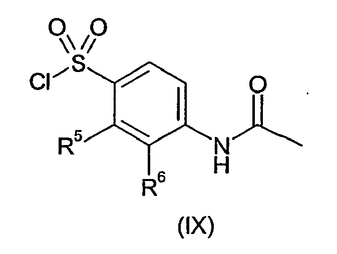 wherein R⁵ and R⁶ are defined above, by treatment with an amine of formula R³R⁴NH, wherein R³ and R⁴ are as defined above, and a suitable base such as sodium acetate, in a suitable solvent such as ethanol, with a suitable amine, at a suitable temperature such as 0°C.

Compounds of formula (IX), wherein R⁵ and R⁶ are as defined above, are either known compounds (for example available from commercial suppliers such as Aldrich) or may be prepared by conventional means. The compound of formula (IX) wherein R⁵ and R⁶ are hydrogen is commercially available (Fluka).

Compounds of formula (II), wherein R³, R⁴, R⁵, R⁶ and X are as defined above may alternatively be prepared from compounds of formula (X); 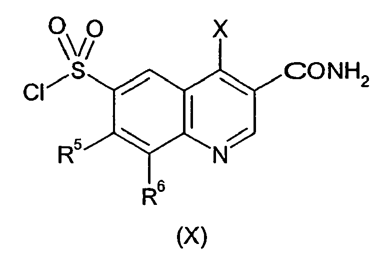 wherein R⁵, R⁶ and X are as defined above, by treatment with an amine of formula R³R⁴NH wherein R³ and R⁴ are as defined above. Suitable conditions include stirring in a suitable solvent such as dichloromethane, or a mixture of dichloromethane and N,N-dimethylformamide, at a suitable temperature such as between 0°C and 20°C in the presence of a suitable base such as *N,N*-diisopropylethylamine.

Compounds of formula (X), wherein R⁵, R⁶ and X are as defined above, may be prepared from compounds of formula (XI); 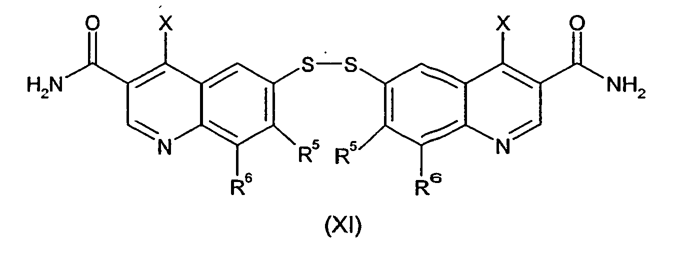 wherein R⁵, R⁶ and X are defined above, by treatment with chlorine. Suitable conditions include stirring in a suitable solvent such as aqueous acetic acid, at a suitable temperature, such as 20°C.

Compounds of formula (XI) wherein R⁵, R⁶ and X are as defined above, may be prepared from compounds of formula (XII); 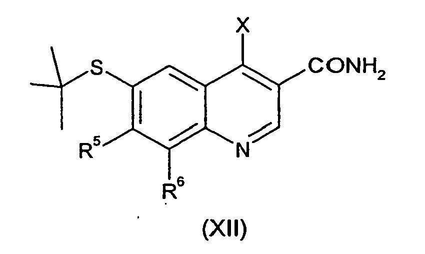 wherein R⁵, R⁶ and X are defined above, by treatment with a suitable acid, such as trifluoroacetic acid, in the presence of a suitable oxidising agent, such as phenyl sulphoxide and a suitable silane, such as methyltrichlorosilane, at a suitable temperature such as 0°C.

Compounds of formula (XII) wherein R⁵, R⁶ and X are as defined above, may be prepared from compounds of formula (XIII): 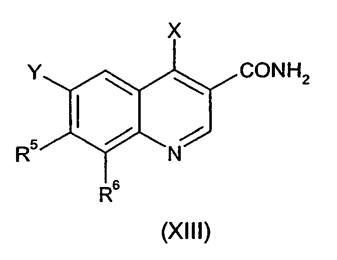 wherein R⁵, R⁶, and X are as defined above and Y is iodine or bromine, by treatment with a suitable tin compound such as *(tert-*butylsulphanyl)tributyltin in a suitable solvent such as toluene, at a suitable temperature, such as 60-120°C, for example 110°C, in the presence of a suitable catalyst such as a palladium catalyst, for example tetrakistriphenylphosphine palladium (0).

The compounds of formula (XIII) may be prepared according to the following synthetic scheme, wherein R⁵, R⁶, X and Y are as defined above:

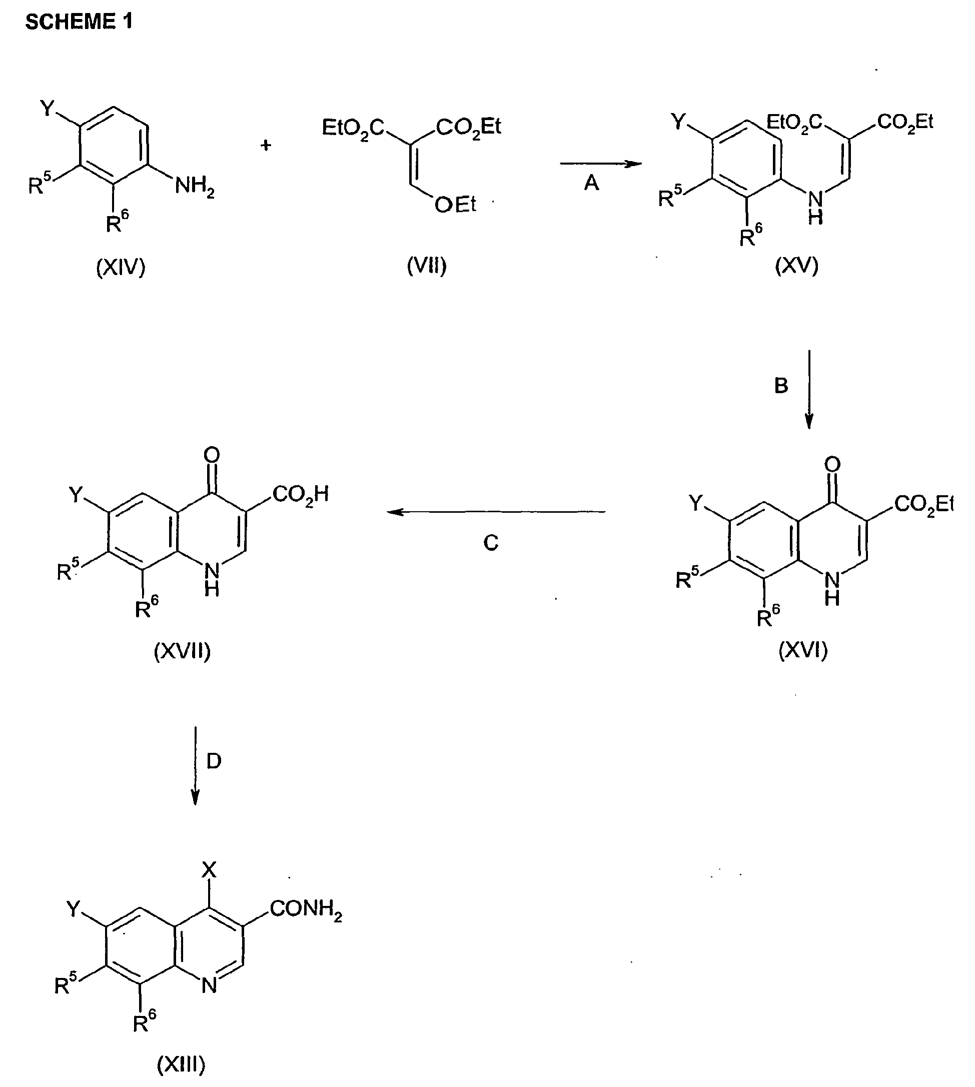

Suitable conditions for the reactions of Scheme 1 are: (A) heating together compounds of formulae (XIV) and (VII) in the absence of solvent, at a suitable temperature, such as 60-100°C, for example at 80°C; (B) heating compounds of formula (XV) in a suitable solvent, such as diphenyl ether, at a suitable temperature such as 200-300°C, for example at 250°C; (C) hydrolysis of compounds of formula (XVI) with a suitable base, such as aqueous sodium hydroxide, in a suitable solvent, such as ethanol, at a suitable temperature such as room temperature; (D) treatment of compounds of formula (XVII) with a suitable halogenating agent, such as a chlorinating agent, for example thionyl chloride, in the presence of a suitable catalyst such as *N,N*-dimethylformamide, followed by treatment with ammonia under suitable conditions, such as 880 ammonia at room temperature.

Preparation of the compounds of formulae (XV) and (XVI) wherein Y represents iodine and R⁵ and R⁶ both represent hydrogen have been previously described in: Indian Journal of Chemistry, Section B: Organic Chemistry Including Medicinal Chemistry (2002), 41B(3), 650-652. Preparation of the compound of formula (XVII) wherein Y represents iodine and R⁵ and R⁶ both represent hydrogen has been previously described in: PCT Int. Appl. (1999), WO 9932450 A1.

Compounds of formulae (XIV), R¹R²NH and R³R⁴NH , wherein R¹, R², R³, R⁴, R⁵, R⁶ and Y are as defined above, are either known compounds (for example available from commercial suppliers such as Aldrich) or may be prepared by conventional means.

Compounds of formulae R¹R²NH and R³R⁴NH may contain amine or acid groups which are suitably protected. Examples of suitable protecting groups and the means for their removal can be found in T. W. Greene and P. G. M. Wuts 'Protective Groups in Organic Synthesis' (3rd Ed., J. Wiley and Sons, 1999). Addition or removal of such protecting groups may be accomplished at any suitable stage in the synthesis of compounds of formula (I).

### Process b

Compounds of formula (I) may also be prepared by a process of interconversion between compounds of formula (I). Processes of interconversion between compounds of formula (I) may include, for example oxidation, reduction, alkylation, dealkylation, acylation, sulfonylation or substitution.

### Process c

Compounds of formula (I) may also be prepared by a process of deprotection of protected derivatives of compounds of formula (I). Examples of suitable protecting groups and the means for their removal can be found in T. W. Greene and P. G. M. Wuts 'Protective Groups in Organic Synthesis' (3rd Ed., J. Wiley and Sons, 1999).

The present invention also provides a compound of formula (I) or a pharmaceutically acceptable salt thereof for use as an active therapeutic substance in a mammal such as a human. The compound or salt can be for use in the treatment and/or prophylaxis of any of the conditions described herein and/or for use as a phosphodiesterase inhibitor, e.g. for use as a phosphodiesterase 4 (PDE4) inhibitor. "Therapy" may include treatment and/or prophylaxis.

Also provided is the use of a compound of formula (I) or a pharmaceutically acceptable salt thereof in the manufacture of a medicament (e.g. pharmaceutical composition) for the treatment and/or prophylaxis of an inflammatory and/or allergic disease in a mammal such as a human.

Phosphodiesterase 4 inhibitors are believed to be useful in the treatment and/or prophylaxis of a variety of diseases, especially inflammatory and/or allergic diseases, in mammals such as humans, for example: asthma, chronic bronchitis, emphysema, atopic dermatitis, urticaria, allergic rhinitis (seasonal or perennial), vasomotor rhinitis, nasal polyps, allergic conjunctivitis, vernal conjunctivitis, occupational conjunctivitis, infective conjunctivitis, eosinophilic syndromes, eosinophilic granuloma, psoriasis, rheumatoid arthritis, chronic obstructive pulmonary disease (COPD) including chronic bronchitis and emphysema, septic shock, ulcerative colitis, Crohn's disease, reperfusion injury of the myocardium and brain, chronic glomerulonephritis, endotoxic shock, adult respiratory distress syndrome, multiple sclerosis, memory impairment (including Alzheimer's disease) pain or depression.

In the treatment and/or prophylaxis, the inflammatory and/or allergic disease is preferably chronic obstructive pulmonary disease (COPD) including chronic bronchitis and emphysema, asthma, rheumatoid arthritis, allergic rhinitis, atopic dermatitis or psoriasis in a mammal (e.g. human). More preferably, the treatment and/or prophylaxis is of COPD including chronic bronchitis and emphysema, asthma or allergic rhinitis in a mammal (e.g. human). PDE4 inhibitors are thought to be effective in the treatment of asthma (e.g. see M.A.Giembycz, Drugs, Feb. 2000, 59(2), 193-212; Z. Huang et al., Current Opinion in Chemical Biology, 2001, 5, 432-438; and refs cited therein) and COPD (e.g. see S.L. Wolda, Emerging Drugs, 2000, 5(3), 309-319; Z. Huang et al., Current Opinion in Chemical Biology, 2001, 5, 432-438; and refs cited therein). COPD is often characterised by the presence of airflow obstruction due to chronic bronchitis and/or emphysema (S.L. Wolda, Emerging Drugs, 2000, 5(3), 309-319).

PDE4 inhibitors are thought to be effective in the treatment of allergic rhinitis (e.g. see B.M. Schmidt et al., J. Allergy & Clinical Immunology, 108(4), 2001, 530-536).

PDE4 inhibitors are thought to be effective in the treatment of rheumatoid arthritis and multiple sclerosis (e.g. see H.J.Dyke et al., Expert Opinion on Investigational Drugs, January 2002, 11 (1), 1-13; C.Burnouf et al., Current Pharmaceutical Design, 2002, 8(14), 1255-1296; and A.M.Doherty, Current Opinion Chem. Biol., 1999, 3(4), 466-473; and refs cited therein). See e.g. A.M.Doherty, Current Opinion Chem. Biol., 1999, 3(4), 466-473 and refs cited therein for atopic dermatitis use.

PDE4 inhibitors have been suggested as having analgesic properties and thus being effective in the treatment of pain (A.Kumar et al., Indian J. Exp. Biol., 2000, 38(1), 26-30).

In the invention, the treatment and/or prophylaxis can be of cognitive impairment e.g. cognitive impairment in a neurological disorder such as Alzheimer's disease. For example, the treatment and/or prophylaxis can comprise cognitive enhancement e.g. in a neurological disorder. See for example: H.T.Zhang et al. in: Psychopharmacology, June 2000, 150(3), 311-316 and Neuropsychopharmacology, 2000, 23(2), 198-204; and T. Egawa et al., Japanese J. Pharmacol., 1997, 75(3), 275-81.

PDE4 inhibitors such as rolipram have been suggested as having antidepressant properties (e.g. J. Zhu et al., CNS Drug Reviews, 2001, 7(4), 387-398; O'Donnell, Expert Opinion on Investigational Drugs, 2000, 9(3), 621-625; and H.T. Zhang et al., Neuropsychopharmacology, October 2002, 27(4), 587-595).

For use in medicine, the compounds of the present invention are usually administered as a pharmaceutical composition.

The present invention therefore provides in a further aspect a pharmaceutical composition comprising a compound of formula (I) or a pharmaceutically acceptable salt thereof and one or more pharmaceutically acceptable carriers and/or excipients.

The pharmaceutical composition can be for use in the treatment and/or prophylaxis of any of the conditions described herein.

The compounds of formula (I) and/or the pharmaceutical composition may be administered, for example, by oral, parenteral (e.g. intravenous, subcutaneous, or intramuscular), inhaled, nasal, transdermal or rectal administration, or as topical treatments (e.g. lotions, solutions, creams, ointments or gels). Accordingly, the pharmaceutical composition is preferably suitable for oral, parenteral (e.g. intravenous, subcutaneous or intramuscular), topical, inhaled or nasal administration. More preferably, the pharmaceutical composition is suitable for topical, inhaled or oral administration, e.g. to a mammal such as a human. Inhaled administration involves topical administration to the lung, e.g. by aerosol or dry powder composition.

A pharmaceutical composition suitable for oral administration can be liquid or solid; for example it can be a solution, a syrup, a suspension or emulsion, a tablet, a capsule or a lozenge.

A liquid formulation will generally consist of a suspension or solution of the compound or pharmaceutically acceptable salt in a suitable pharmaceutically acceptable liquid carrier(s), for example an aqueous solvent such as water, aqueous ethanol or aqueous glycerine, or an oil, or a non-aqueous solvent, such as a surfactant, such as polyethylene glycol. The formulation may also contain a suspending agent, preservative, flavouring and/or colouring agent.

A pharmaceutical composition suitable for oral administration being a tablet can comprise one or more pharmaceutically acceptable carriers and/or excipients suitable for preparing tablet formulations. Examples of such carriers include lactose and cellulose. The tablet can also or instead contain one or more pharmaceutically acceptable excipients, for example binding agents, lubricants such as magnesium stearate, and/or tablet disintegrants.

A pharmaceutical composition suitable for oral administration being a capsule can be prepared using encapsulation procedures. For example, pellets containing the active ingredient can be prepared using a suitable pharmaceutically acceptable carrier and then filled into a hard gelatin capsule. Alternatively, a dispersion, suspension or solution can be prepared using any suitable pharmaceutically acceptable carrier, for example an aqueous solution, aqueous gum or an oil and the dispersion, suspension or solution then filled into a soft or hard gelatin capsule.

The compounds of formula (I) and/or the pharmaceutical composition may be administered by a controlled or sustained release formulation as described in WO 00/50011.

A parenteral composition can comprise a solution or suspension of the compound or pharmaceutically acceptable salt in a sterile aqueous carrier or parenterally acceptable oil.

Alternatively, the solution can be lyophilised; the lyophilised parenteral pharmaceutical composition can be reconstituted with a suitable solvent just prior to administration.

Compositions for nasal or inhaled administration may conveniently be formulated as aerosols, solutions, drops, gels or dry powders.

For compositions suitable and/or adapted for inhaled administration, it is preferred that the compound or salt of formula (I) is in a particle-size-reduced form, and more preferably the size-reduced form is obtained or obtainable by micronisation. The preferable particle size of the size-reduced (e.g. micronised) compound or salt is defined by a D50 value of about 0.5 to about 10 microns (for example as measured using laser diffraction).

Aerosol formulations, e.g. for inhaled administration, can comprise a solution or fine suspension of the active substance in a pharmaceutically acceptable aqueous or non-aqueous solvent. Aerosol formulations can be presented in single or multidose quantities in sterile form in a sealed container, which can take the form of a cartridge or refill for use with an atomising device or inhaler. Alternatively the sealed container may be a unitary dispensing device such as a single dose nasal inhaler or an aerosol dispenser fitted with a metering valve (metered dose inhaler) which is intended for disposal once the contents of the container have been exhausted.

Where the dosage form comprises an aerosol dispenser, it preferably contains a suitable propellant under pressure such as compressed air, carbon dioxide or an organic propellant such as a hydrofluorocarbon (HFC). Suitable HFC propellants include 1,1,1,2,3,3,3-heptafluoropropane and 1,1,1,2-tetrafluoroethane. The aerosol dosage forms can also take the form of a pump-atomiser. The pressurised aerosol may contain a solution or a suspension of the active compound. This may require the incorporation of additional excipients e.g. co-solvents and/or surfactants to improve the dispersion characteristics and homogeneity of suspension formulations. Solution formulations may also require the addition of co-solvents such as ethanol. Other excipient modifiers may also be incorporated to improve, for example, the stability and/or taste and/or fine particle mass characteristics (amount and/or profile) of the formulation.

For pharmaceutical compositions suitable and/or adapted for inhaled administration, it is preferred that the pharmaceutical composition is a dry powder inhalable composition. Such a composition can comprise a powder base such as lactose, glucose, trehalose, mannitol or starch, the compound of formula (I) or salt thereof (preferably in particle-size-reduced form, e.g. in micronised form), and optionally a performance modifier such as L-leucine or another amino acid, cellobiose octaacetate and/or metals salts of stearic acid such as magnesium or calcium stearate. Preferably, the dry powder inhalable composition comprises a dry powder blend of lactose and the compound of formula (I) or salt thereof. The lactose is preferably lactose hydrate e.g. lactose monohydrate and/or is preferably inhalation-grade and/or fine-grade lactose. Preferably, the particle size of the lactose is defined by 90% or more (by weight or by volume) of the lactose particles being less than 1000 microns (micrometres) (e.g. 10-1000 microns e.g. 30-1000 microns) in diameter, and/or 50% or more of the lactose particles being less than 500 microns (e.g. 10-500 microns) in diameter. More preferably, the particle size of the lactose is defined by 90% or more of the lactose particles being less than 300 microns (e.g. 10-300 microns e.g. 50-300 microns) in diameter, and/or 50% or more of the lactose particles being less than 100 microns in diameter. Optionally, the particle size of the lactose is defined by 90% or more of the lactose particles being less than 100-200 microns in diameter, and/or 50% or more of the lactose particles being less than 40-70 microns in diameter. Most importantly, it is preferable that about 3 to about 30% (e.g. about 10%) (by weight or by volume) of the particles are less than 50 microns or less than 20 microns in diameter. For example, without limitation, a suitable inhalation-grade lactose is E9334 lactose (10% fines) (Borculo Domo Ingredients, Hanzeplein 25, 8017 JD Zwolle, Netherlands).

Optionally, in particular for dry powder inhalable compositions, a pharmaceutical composition for inhaled administration can be incorporated into a plurality of sealed dose containers (e.g. containing the dry powder composition) mounted longitudinally in a strip or ribbon inside a suitable inhalation device. The container is rupturable or peel-openable on demand and the dose of e.g. the dry powder composition can be administered by inhalation via the device such as the DISKUS ™ device, marketed by GlaxosmithKline. The DISKUS ™ inhalation device is for example described in GB 2242134 A, and in such a device at least one container for the pharmaceutical composition in powder form (the container or containers preferably being a plurality of sealed dose containers mounted longitudinally in a strip or ribbon) is defined between two members peelably secured to one another; the device comprises: a means of defining an opening station for the said container or containers; a means for peeling the members apart at the opening station to open the container; and an outlet, communicating with the opened container, through which a user can inhale the pharmaceutical composition in powder form from the opened container.

For application topically to the skin, the compound of formula (I) or a pharmaceutically acceptable salt thereof could be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, it could be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

In the pharmaceutical composition, each dosage unit for oral or parenteral administration preferably contains from 0.01 to 3000 mg, more preferably 0.5 to 1000 mg, of a compound of the formula (I) or a pharmaceutically acceptable salt thereof, calculated as the free base. Each dosage unit for nasal or inhaled administration preferably contains from 0.001 to 50 mg, more preferably 0.005 to 5 mg, of a compound of the formula (I) or a pharmaceutically acceptable salt thereof, calculated as the free base.

The pharmaceutically acceptable compounds or salts of the invention can be administered in a daily dose (for an adult patient) of, for example, an oral or parenteral dose of 0.01 mg to 3000 mg per day or 0.5 to 1000 mg per day, or a nasal or inhaled dose of 0.001 to 50 mg per day or 0.005 to 5 mg per day, of the compound of the formula (I) or a pharmaceutically acceptable salt thereof, calculated as the free base.

The compounds, salts and/or pharmaceutical compositions according to the invention may also be used in combination with one or more other therapeutically active agents, for example, a β₂ adrenoreceptor agonist, an anti-histamine, an anti-allergic agent, an anti-inflammatory agent (including a steroid), an anticholinergic agent or an antiinfective agent (e.g. antibiotics or antivirals).

The invention thus provides, in a further aspect, a combination comprising a compound of formula (I) or a pharmaceutically acceptable salt thereof with one or more other therapeutically active agents, for example, a β₂-adrenoreceptor agonist, an anti-histamine, an anti-allergic agent, an anti-inflammatory agent (including a steroid), an anticholinergic agent or an antiinfective agent (e.g. antibiotics or antivirals).

Examples of β₂-adrenoreceptor agonists include salmeterol (e.g. as racemate or a single enantiomer such as the R-enantiomer), salbutamol, formoterol, salmefamol, fenoterol or terbutaline and salts thereof, for example the xinafoate salt of salmeterol, the sulphate salt or free base of salbutamol or the fumarate salt of formoterol. Long-acting β₂-adrenoreceptor agonists are preferred, especially those having a therapeutic effect over a 24 hour period such as salmeterol or formoterol.

Examples of anti-histamines include methapyrilene, loratadine, cetirizine, desloratadine or fexofenadine.

Examples of anti-inflammatory steroids include fluticasone propionate and budesonide.

Examples of anticholinergic compounds which may be used in combination with a compound of formula (I) or a pharmaceutically acceptable salt thereof are described in WO 03/011274 A2 and WO 02/069945 A2 / US 2002/0193393 A1 and US 2002/052312 A1. For example, anticholinergic agents include muscarinic M3 antagonists, such as ipratropium bromide, oxitropium bromide or tiotropium bromide.

Other suitable combinations include, for example, combinations comprising a compound of formula (I) or a pharmaceutically acceptable salt thereof together with other anti-inflammatory agents (e.g. anti-inflammatory corticosteroids, NSAID_{S}, leukotriene antagonists (e.g. montelukast), iNOS inhibitors, tryptase and elastase inhibitors, beta-2 integrin antagonists, chemokine antagonists such as CCR3 antagonists, adenosine 2a agonists, 5-lipoxygenase inhibitors and antiinfective agents such as an antibiotic or an antiviral). An iNOS inhibitor is preferably for oral administration. Suitable iNOS inhibitors (inducible nitric oxide synthase inhibitors) include those disclosed in WO 93/13055, WO 98/30537, WO 02/50021, WO 95/34534 and WO 99/62875. Suitable CCR3 inhibitors include those disclosed in WO 02/26722.

The combinations referred to above may conveniently be presented for use in the form of a pharmaceutical composition and thus a pharmaceutical composition comprising a combination as defined above together with one or more pharmaceutically acceptable carriers and/or excipients represent a further aspect of the invention.

The individual compounds of such combinations may be administered either sequentially or simultaneously in separate or combined pharmaceutical compositions.

### Biological Test Methods

### PDE3, PDE4B, PDE4D, PDE5 Primary assay methods

The activity of the compounds can be measured as described below. Preferred compounds of the invention are selective PDE4 inhibitors, i.e. they inhibit PDE4 (e.g. PDE4B and/or PDE4D) more strongly than they inhibit other PDE's such as PDE3 and/or PDE5.

### PDE enzyme sources and literature references

Human recombinant PDE4B, in particular the 2B splice variant thereof (HSPDE4B2B), is disclosed in WO 94/20079 and also in M.M. McLaughlin et al., "A low Km, rolipram-sensitive, cAMP-specific phosphodiesterase from human brain: cloning and expression of cDNA, biochemical characterisation of recombinant protein, and tissue distribution of mRNA", J. Biol. Chem., 1993, 268, 6470-6476. For example, in Example 1 of WO 94/20079, human recombinant PDE4B is described as being expressed in the PDE-deficient yeast *Saccharomyces cerevisiae* strain GL62, e.g. after induction by addition of 150 uM CuSO₄, and 100,000 x g supernatant fractions of yeast cell lysates are described for use in the harvesting of PDE4B enzyme.

Human recombinant PDE4D (HSPDE4D3A) is disclosed in P. A. Baecker et al., "Isolation of a cDNA encoding a human rolipram-sensitive cyclic AMP phoshodiesterase (PDE IVD)", Gene, 1994, 138, 253-256.

Human recombinant PDE5 is disclosed in K. Loughney et al., "Isolation and characterisation of cDNAs encoding PDE5A, a human cGMP-binding, cGMP-specific 3',5'-cyclic nucleotide phosphodiesterase", Gene, 1998, 216, 139-147. '

PDE3 may be purified from bovine aorta as described by H. Coste and P. Grondin, "Characterisation of a novel potent and specific inhibitor of type V phosphodiesterase", Biochem. Pharmacol., 1995, 50, 1577-1585.

PDE6 may be purified from bovine retina as described by: P. Catty and P. Deterre, "Activation and solubilization of the retinal cGMP-specific phosphodiesterase by limited proteolysis", Eur. J. Biochem., 1991, 199, 263-269; A. Tar et al. "Purification of bovine retinal cGMP phosphodiesterase", Methods in Enzymology, 1994, 238, 3-12; and/or D. Srivastava et al. "Effects of magnesium on cyclic GMP hydrolysis by the bovine retinal rod cyclic GMP phosphodiesterase", Biochem. J., 1995, 308, 653-658.

### Inhibition of PDE3, PDE4B,PDE 4D, PDE5 or PDE 6 activity: radioactive Scintillation Proximity Assay (SPA)

The ability of compounds to inhibit catalytic activity at PDE4B or 4D (human recombinant), PDE3 (from bovine aorta) PDE5 (human recombinant) or PDE 6 (from bovine retina) may be determined by Scintillation Proximity Assay (SPA) in 96-well format. Test compounds (preferably as a solution in DMSO, e.g. 2 microlitre (µl) volume) were preincubated at ambient temperature in Wallac Isoplates (code 1450-514) with PDE enzyme in 50mM Tris-HCl buffer pH 7.5 , 8.3mM MgCl₂, 1.7mM EGTA, 0.05% (w/v) bovine serum albumin for 10-30 minutes. The enzyme concentration was adjusted so that control rates are linear over the asay incubation period. For the PDE3, PDE4B and PDE4D assays [5',8-³H]adenosine 3',5'-cyclic phosphate (Amersham Pharmacia Biotech , code TRK559 or Amersham Biosciences UK Ltd, Pollards Wood, Chalfont St Giles, Buckinghamshire HP8 4SP, UK) was added to give 0.05µCi per well and ~ 10nM final concentration. For the PDE5 and PDE6 assays [8-³H]guanosine 3',5'-cyclic phosphate (Amersham Pharmacia Biotech , code TRK392) was added to give 0.05µCi per well and - 36nM final concentration. Plates e.g. containing approx. 100 µl volume of assay mixture were mixed on an orbital shaker for 5 minutes and incubated at ambient temperature for 1 hour. Phosphodiesterase SPA beads (Amersham Pharmacia Biotech, code RPNQ0150) were added (~1 mg per well) to terminate the assay. Plates were sealed and shaken and allowed to stand at ambient temperature for 35 minutes to 1 hour to allow the beads to settle. Bound radioactive product was measured using a WALLAC TRILUX 1450 MicroBeta scintillation counter. For inhibition curves, 10 concentrations (e.g. 1.5nM - 30µM) of each compound were assayed; more potent compounds were assayed over lower concentration ranges (assay concentrations were generally between 30µM and 50fM). Curves were analysed using ActivityBase and XLfit (lD Businesss Solutions Limited, 2 Ocean Court, Surrey Research Park, Guildford, Surrey GU2 7QB, United Kindgom). Results were expressed as plC₅₀ values.

Alternatively, the activity of the compounds can be measured in the following Fluorescence Polarisation (FP) assay:
*Inhibition* of *PDE4B or PDE4D activity: Fluorescence Polarisation (FP) assay*

The ability of compounds to inhibit catalytic activity at PDE4B (human recombinant) and PDE4D (human recombinant) was determined by IMAP Fluorescence Polarisation (FP) assay (Molecular Devices Ltd code: R8062) in 384-well format. Test compounds (small volume, e.g. 0.5 µl, of solution in DMSO) were preincubated at ambient temperature in black 384-well microtitre plates (supplier: NUNC, code 262260) with PDE enzyme in 10mM Tris-HCl buffer pH 7.2, 10mM. MgCl₂, 0.1 % (w/v) bovine serum albumin, 0.05% NaN₃ for 10-30 minutes. The enzyme level was set so that reaction was linear throughout the incubation.

Fluorescein adenosine 3',5'-cyclic phosphate (Molecular Devices Ltd code: R7091) was added to give - 40nM final concentration. Plates were mixed on an orbital shaker for 10 seconds and incubated at ambient temperature for 40 minutes. IMAP binding reagent (Molecular Devices Ltd code: R7207) was added (60µl of a 1 in 400 dilution in binding buffer of the kit stock solution) to terminate the assay. Plates were allowed to stand at ambient temperature for 1 hour. The FP ratio of parallel to perpendicular light was measured using an Analyst^{™} plate reader (from Molecular Devices Ltd). For inhibition curves, 11 concentrations (0.5nM - 30µM) of each compound were assayed; more potent compounds were assayed over lower concentration ranges (assay concentrations were generally between 30µM and 50fM). Curves were analysed using ActivityBase and XLfit (ID Businesss Solutions Limited). Results were expressed as plC₅₀ values.

For a given PDE4 inhibitor, the PDE4B (or PDE4D) inhibition values measured using the SPA and FP assays can differ slightly. However, in a regression analysis of at least 100 test compounds, the pIC₅₀ inhibition values measured using SPA and FP assays have been found generally to agree within 0.5 log units, for PDE4B and PDE4D (linear regression coefficient 0.966 for PDE4B and 0.971 for PDE4D; David R.Mobbs et al., "Comparison of the IMAP Fluorescence Polarisation Assay with the Scintillation Proximity Assay for Phosphodiesterase Activity", poster presented at the 2003 Molecular Devices UK & Europe User Meeting, 2nd October 2003, Down Hall, Harlow, Essex, United Kingdom).

Examples of compounds of the invention described above inhibit the catalytic activity at the PDE4B (human recombinant) enzyme with plC₅₀'s in the range 7.5-10.8. Biological Data obtained for some of the Examples (PDE4B and PDE5 inhibitory activity) is as follows:

| **Example No.** | **PDE4B mean pIC₅₀** | **PDE5 mean PIC₅₀** |
|---|---|---|
| **2** | 9.0 | 4.8 |
| **4** | 8.6 | 4.9 |
| **9** | 8.2 | 4.6 |

*Emesis:* Many known PDE4 inhibitors cause emesis and/or nausea to greater or lesser extents (e.g. see Z. Huang et al., Current Opinion in Chemical Biology, 2001, 5, 432-438, see especially pages 433-434 and refs cited therein). Therefore, it would be preferable but not essential that a PDE4 inhibitory compound of the invention causes only limited or manageable emetic side-effects. Emetic side-effects can for example be measured by the emetogenic potential of the compound when administered to ferrets; for example one can measure the time to onset, extent, frequency and/or duration of vomiting and/or writhing in ferrets after oral or parenteral administration of the compound. See for example A. Robichaud et al., "Emesis induced by inhibitors of PDE IV in the ferret" Neuropharmacology, 1999, 38, 289-297, erratum Neuropharmacology, 2001, 40, 465-465.

All publications, including but not limited to patents and patent applications, cited in this specification are herein incorporated by reference as if each individual publication were specifically and individually indicated to be incorporated by reference herein as though fully set forth.

### EXAMPLES

The various aspects of the invention will now be described by reference to the following examples. These examples are merely illustrative and are not to be construed as a limitation of the scope of the present invention. In this section, "intermediates" represent syntheses of intermediate compounds intended for use in the synthesis of the "examples"

Abbreviations used herein:

| | |
|---|---|
| HPLC | high performance liquid chromatography |
| NMR | nuclear magnetic resonance |
| LC/MS | liquid chromatography/mass spectroscopy |
| TLC | thin layer chromatography |
| SPE | solid phase extraction column. Unless otherwise specified the solid phase will be silica gel. Aminopropyl SPE refers to a silica SPE column with aminopropyl residues immobilised on the solid phase (eg. IST Isolute^{™} columns). It is thought that compounds isolated by SPE are free bases. |
| SCX | solid phase extraction (SPE) column with benzene sulfonic acid residues immobilised on the solid phase (eg. IST Isolute^{™} columns). When eluting with ammonia/ methanol, it is thought that compounds isolated by SCX are free bases. |

### General experimental details

LC/MS (liquid chromatography/mass spectroscopy)
   Waters ZQ mass spectrometer operating in positive ion electrospray mode, mass range 100-1000 amu.
   UV wavelength : 215-330nM
   Column : 3.3cm x 4.6mm ID, 3µm ABZ+PLUS
   Flow Rate: 3ml/min
   Injection Volume : 5µl
   Solvent A : 95% acetonitrile + 0.05% formic acid
   Solvent B : 0.1 % formic acid + 10mM ammonium acetate
   Gradient: Mixtures of Solvent A and Solvent B are used according to the following gradient profiles (expressed as % Solvent A in the mixture): 0% A/0.7min, 0-100%
   A/3.5min, 100% A/1.1 min; 100-0% A/0.2min
Mass Directed Automated Preparative HPLC column, conditions and eluent
   Method A
      The preparative column used was a Supelcosil ABZplus (10cm x 2.12cm internal diameter; particle size 5µm)
      UV detection wavelength : 200-320nM
      Flow rate: 20ml/min
      Injection Volume: 0.5ml
      Solvent A : 0.1% formic acid
      Solvent B : 95% acetonitrile + 0.05% formic acid
      Gradient systems: mixtures of Solvent A and Solvent B are used according to a choice of 5 generic gradient profiles (expressed as % Solvent B in the mixture), ranging from a start of 0 to 50% Solvent B, with all finishing at 100% Solvent B to ensure total elution.
         It is thought that compounds isolated by this method are free bases, unless the R¹ or R³ groups contain basic moieties, in which case formate salts may be formed.
Mass Directed Automated Preparative HPLC column, conditions and eluent
   Method B
      The preparative column used was a Supelcosil ABZplus (10cm x 2.12cm internal diameter; particle size 5µm)
      UV detection wavelength : 200-320nM
      Flow rate : 20ml/min
      Injection Volume: 0.5ml
      Solvent A : water + 0.1 % trifluoroacetic acid
      Solvent B : acetonitrile + 0.1 % trifluoroacetic acid
      Gradient systems: mixtures of Solvent A and Solvent B are used according to a choice of 5 generic gradient profiles (expressed as % Solvent B in the mixture), ranging from a start of 0 to 50% Solvent B, with all finishing at 100% Solvent B to ensure total elution.

It is thought that compounds isolated by this method are trifluoroacetate salts.

### Product isolation by filtration directly from the reaction mixture

It is thought that compounds isolated by this method from reactions involving displacement of a 4-chloroquinoline intermediate with an amine of formula R¹R²NH are hydrochloride salts.

### 'Hydrophobic Frit'

This refers to a Whatman PTFE filter medium (frit), pore size 5.0µm, housed in a polypropylene tube.

### Evaporation of product fractions after purification

Reference to column chromatography, SPE and preparative HPLC purification includes evaporation of the product containing fractions to dryness by an appropriate method.

### Aqueous ammonia solutions

'880 Ammonia' or '0.880 ammonia' refers to concentrated aqueous ammonia (specific gravity 0.880).

### Intermediates and Examples

All reagents not detailed in the text below are commercially available from established suppliers such as Sigma-Aldrich.

### Intermediate 1. Diethyl ({[4-(1-piperidinyisulfonyl)phenyl]amino}methylidene) propanedioate

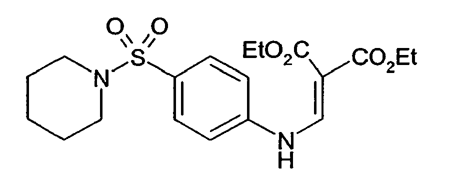

4-(1-Piperidinylsulfonyl)aniline (0.20g) (available from Maybridge International) and diethyl (ethoxymethylene)malonate (0.18g) (available from Aldrich) were heated at 150°C under 150W microwave irradiation for 15mins. The mixture was diluted with cyclohexane, filtered and the residue dried at 40°C *in vacuo* to give the title compound as a pale pink solid (0.284g).
LC/MS Rt 3.36min m/z 411 [MH⁺]

### Intermediate 2. Ethyl 4-oxo-6-(1-piperidinylsulfonyl)-1,4-dihydro-3-quinolinecarboxylate

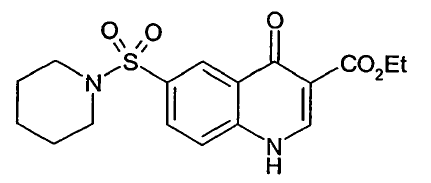

Intermediate 1 (0.284g) was suspended in diphenyl ether (15ml) and heated at 250°C for 2h. After cooling, the mixture was diluted with cyclohexane (50ml) and the resulting precipitate filtered off and dried *in vacuo* to give the title compound as a brown solid (0.138g):
LC/MS Rt 2.68min *m*/*z* 365 [MH⁺]

### Intermediate 3. 4-Oxo-6-(1-piperidinylsulfonyl)-1.4-dihydro-3-quinolinecarboxylic acid

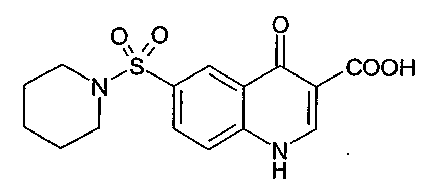

Intermediate 2 (0.138g) was dissolved in ethanol (2ml) and 2M sodium hydroxide (2ml) and the mixture heated under reflux for 3h. After cooling the solvent was removed under a stream of nitrogen and the residue dissolved in water (2ml) and extracted with ethyl acetate (2 x 4ml). The aqueous layer was acidified to pH 6.0 using 2M hydrochloric acid, and the resulting precipitate removed by filtration and dried *in vacuo* at 40°C to give the title compound (0.052g).
LC/MS Rt 2.83min *m*/*z* 337 [MH⁺]

### Intermediate 4. 4-Chloro-6-(1-piperidinylsulfonyl)-3-quinolinecarboxamide

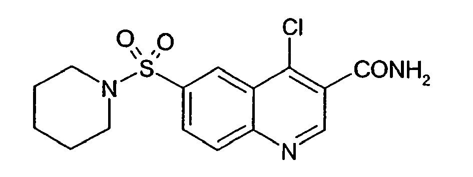

Intermediate 3 (0.051g) was suspended in thionyl chloride (4ml) and treated with N,N-dimethylformamide (4 drops), and the mixture heated at 80°C for 18h. The solvent was removed *in vacuo* and the residue azeotroped with toluene (5ml). The resulting solid was added to 880 ammonia (4ml) and the mixture stirred at room temperature for 3h. The solid was removed by filtration to give the title compound as a brown solid (0.011g). LC/MS Rt 2.63min *m*/*z* 354 [MH⁺]

### Intermediate 5. Diethyl {[(4-iodophenyl)amino]methylidene}propanedioate

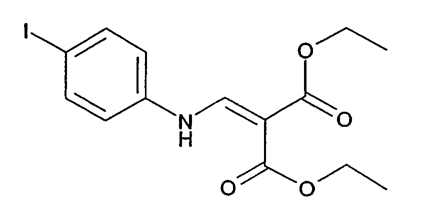

A mixture of 4-iodoaniline (208g) (available from Aldrich) and diethyl (ethoxymethylene)malonate (210ml) (available from Aldrich) was heated to 100°C. The mixture set solid at ca. 60°C, and was removed from heating and broken up. Heating was continued at 100°C for 1h, and the solid was collected, washed with cyclohexane (1000ml) and ethanol (2x500ml), and dried *in vacuo* at 40°C overnight to give the title compound as a white solid (356g).
LC/MS Rt 3.57.min m/z 390 [MH⁺].

### Intermediate 6. Ethyl 6-iodo-4-oxo-1,4-dihydro-3-quinolinecarboxylate

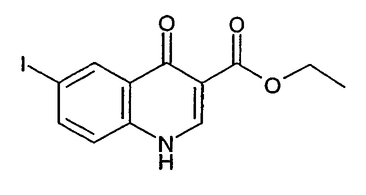

Diphenyl ether (170ml) was heated to reflux and intermediate 5 (30g) was gradually added down an air condenser. Once all the reagent had been added the mixture was heated under reflux for a further 30min. The mixture was then cooled and isohexane (200ml) was added. The solid formed was collected by filtration to give the title compound (19.2g).
NMR: (d-6 DMSO) δ 8.58 (1H,s), 8.42(1H,d), 7.99 (1H,dd), 7.44(1H,d), 4.21(2H,q), 1.28 (3H,t).

### Intermediate 7. 6-Iodo-4-oxo-1,4-dihydro-3-quinolinecarboxylic acid

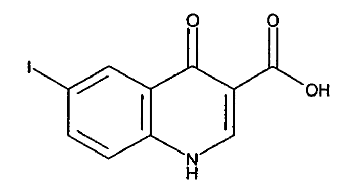

Sodium hydroxide (9.8g) was dissolved in water (61 ml) and ethanol (30ml) was added. The resultant solution was added to intermediate 6 (10.0g), and the mixture was heated under reflux for 60min with stirring under nitrogen. Concentrated hydrochloric acid was added, giving a white precipitate. After stirring for 16h, the precipitate was filtered off, washed with water and dried *in vacuo* to give the title compound as a white solid (8.15g). LC/MS Rt 3.01 min *m*/*z* 316 [MH⁺].

### Intermediate 8. 4-Chloro-6-iodo-3-quinolinecarboxamide

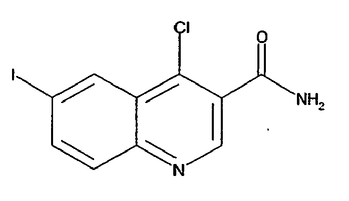

Intermediate 7 (8.1g) was added portionwise to stirred thionyl chloride (60ml). *N,N-*dimethylformamide (3 drops) was added and the mixture was heated under reflux with stirring under nitrogen for 1.75h. The excess thionyl chloride was evaporated *in vacuo* and the residue was azeotroped with toluene (2x50ml). The resulting pale yellow solid was added portionwise to stirred 880 ammonia (250ml), and the mixture stirred at room temperature for 1.5h. The solid was filtered off, washed with water and dried *in* vacuo at 60°C for 16h to give the title compound as a white solid (7.94g).
LC/MS Rt 2.72min *m*/*z* 332 [MH⁺].

### Intermediate 9. 4-Chloro-6-[(1,1-dimethylethyl)thio]-3-quinoline carboxamide

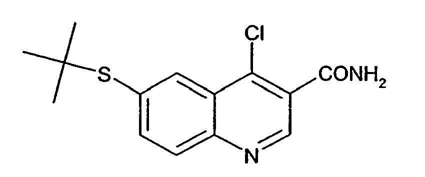

To a stirred mixture of intermediate 8 (14.7g) and tetrakistriphenylphosphine palladium (0) (1.02g) in toluene (250ml) under nitrogen was added a solution of *(tert-*butylsulphanyl)tributyltin *(*JACS 2002,124, 4874) (20.1g) in toluene (50ml), and the mixture was heated under reflux for 1 h. The mixture was cooled to room temperature, partitioned between 5% potassium fluoride solution (1000ml) and diethyl ether (500ml) and the organic solvent evaporated *in vacuo.* The solid obtained was triturated with diethyl ether and filtered to give the title compound as a pale orange solid (9.47g). The filtrate was evaporated *in vacuo.* Purification by chromatography on silica gel, eluting with diethyl ether then ethyl acetate, gave further title compound as an orange solid (2.97g; total yield 12.4g).
LC/MS Rt 3.04min *m*/*z* 295 [MH⁺]

### Intermediate 10. 6,6'-Dithiobis(4-chloro-3-quinolinecarboxamide)

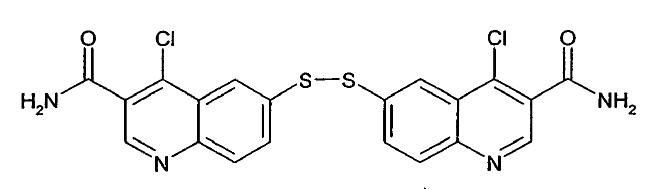

Intermediate 9 (12.3g) was dissolved in trifluoroacetic acid (200ml), phenyl sulphoxide (21.2g) added, and the mixture cooled to 0°C. Methyltrichlorosilane (49ml) was added over 10mins, and the mixture stirred for 1 h. The mixture was evaporated *in vacuo,* the resid ue triturated with diethyl ether (250ml), and the solvent decanted off. The residue was triturated twice more with diethyl ether (200ml) and the solid filtered off to give the title compound as a pale yellow solid (10.5g).
LC/MS Rt 2.87min *m*/*z* 475 [MH⁺]

Similarly prepared using 4-iodo-2-methylaniline instead of 4-iodoaniline (as in the preparation of Intermediate 5) was:

### Intermediate 10a. 6,6'-Dithiobis(4-chloro-8-methyl-3-quinolinecarboxamide)

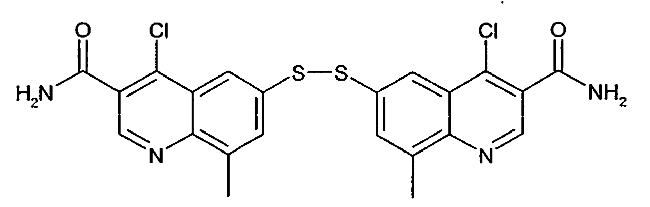

LC/MS Rt 3.50min m/z 503 [MH⁺]

### Intermediate 11. 3-(Aminocarbonyl)-4-chloro-6-puinolinesulfonyl chloride

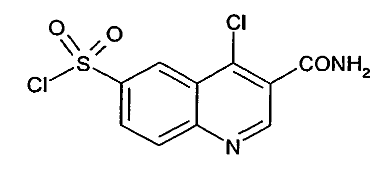

Chlorine was bubbled through a suspension of intermediate 10 (0.20g) in acetic acid (4ml) and water (1ml) for 5min, giving a yellow solution. The mixture was partitioned between water (50ml) and diethyl ether (50ml) and the organic layer dried (Na₂SO₄) and concentrated *in vacuo* to give the title compound as a pale yellow solid (0.248g).
LC/MS Rt 2.63min m/z 305 [MH⁺]

### Intermediate 12. 4-Chloro-6-(4-morpholinylsulfonyl)-3-quinolinecarboxamide

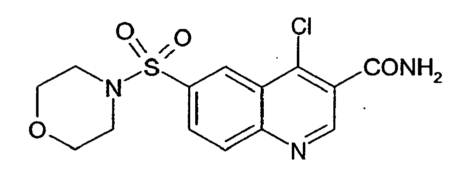

A solution of intermediate 11 (0.88g) in dichloromethane (10ml) and *N,N-*dimethylformamide (5ml) was added to a solution of morpholine (0.131ml) and N,N-diisopropylethylamine (1.31ml) in dichloromethane (30ml) at 0-5°C. The mixture was allowed to warm to 20°C over 18h, diluted with dichloromethane (150ml), and extracted with 1 M hydrochloric acid (100ml) followed by saturated aqueous sodium bicarbonate solution (100ml). The organic layer was dried (Na₂SO₄) and evaporated *in vacuo* to give the title compound as a brown gum (0.571g).
LC/MS Rt 2.22min m/z 356 [MH⁺]

Similarly prepared from intermediate 11 were the following:

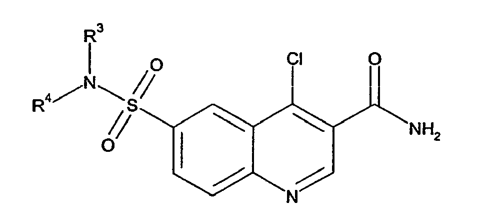

| **Intermediate Number** | **R³R⁴N-** | **Amine Reagent R³R⁴NH/ Source** (a) | **LC/MS Rt min** | **LC/MS MH⁺** |
|---|---|---|---|---|
| 13 | 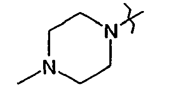 | 1-Methylpiperazine/ Aldrich | 1.76 | 369 |
| 14 | 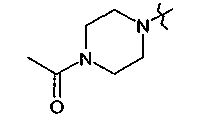 | 1-Acetylpiperazine/ Aldrich | 2.26 | 397 |
| 15 | 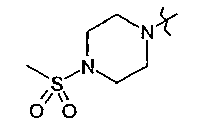 | 1-(Methylsulphonyl) piperazine/ Patent: DE828695 (1950) | 2.38 | 433 |
| 16 | 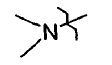 | Dimethylamine.HCl/ Aldrich | 2.25 | 314 |
| 17 | 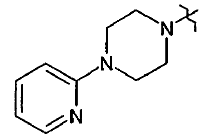 | (2-Pyridinyl) piperazine/Aldrich | 2.24 | 432 |
| 18 | 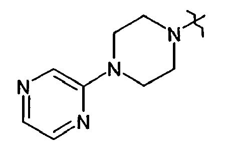 | 2-(1-Piperazinyl) pyrazine/Emkachem | 2.53 | 433 |
| 19 | 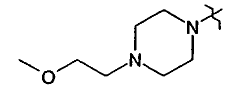 | 1-[2-(Methyloxy) ethyl]piperazine/Em kachem | 1.90 | 413 |
| 20 | 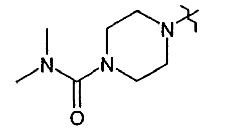 | N,N-Dimethyl-1-piperazine-carboxamide/ Intermediate 27 | 2.37 | 426 |
| 21 | 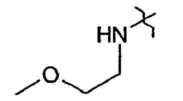 | [2-(methyloxy)-ethyl]amine/Aldrich | 2.10 | 344 |
| 22 | 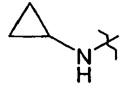 | Cyclopropylamine/ Aldrich | 2.26 | 326 |
| 23 | 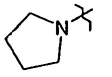 | Cyclopentylamine/ Aldrich | 2.41 | 340 |
| 24 | 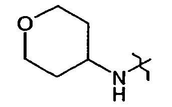 | Tetrahydro-2*H*-pyran-4-ylamine/ Combi- Block | 2.16 | 370 |

| | | | | |
|---|---|---|---|---|
| (a) Where available, a salt such as the hydrochloride salt of the amine R³R⁴NH may be used. | | | | |

### Intermediate 25. 1,1-Dimethylethyl 4-(chlorocarbonyl)-1-piperazinecarboxylate

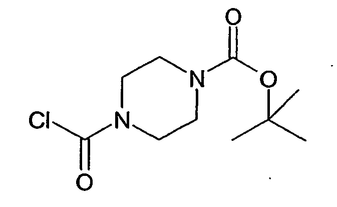

A solution of 1,1-dimethylethyl 1-piperazinecarboxylate (13.0g) (available from Aldrich) and pyridine (11.2ml) in dichloromethane (30ml) was added dropwise to a solution of triphosgene (8.3g) in dichloromethane (60ml) at 0-5°C. The cooling bath was removed and the mixture warmed to room temperature over 30min. The mixture was quenched by the dropwise addition of 1 M hydrochloric acid (50ml). The organic layer was separated and washed successively with 1 M hydrochloric acid (40ml), water (40ml) and saturated sodium chloride solution (40ml), dried (Na₂SO₄) and evaporated *in vacuo* to give the title compound as a yellow solid (16.0g).
¹HNMR (CDCl₃) δ 3.71 (2H,m,CH₂), 3.62 (2H,m,CH₂), 3.5 (4H,m,2xCH₂), 1.5 (9H,s,3xCH₃).

### Intermediate 26. 1,1-Dimethylethyl 4-[(dimethylamino)carbonyl]-1-piperazinecarboxylate

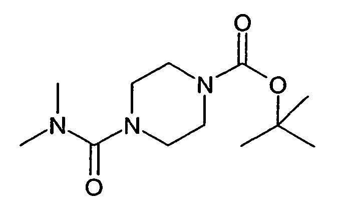

To a solution of dimethylamine hydrochloride (0.15g) (available from Aldrich) in dichloromethane (5ml) was added triethylamine (0.305g) and the mixture stirred for 10min, after which intermediate 25 (0.3g) was added. The mixture was heated under reflux for 3hr, cooled, diluted with dichloromethane (20ml) and washed with water (20ml). The organic layer was dried and evaporated *in vacuo;* the residue was loaded in methanol onto a sulphonic acid ion exchange cartridge (Isolute SCX), and the cartridge was eluted with methanol. Evaporation of the solvent gave the title compound as a white solid (0.276g).
¹HNMR (CDCl₃) δ 3.45 (4H,m,CH₂), 3.2 (4H,m,2xCH₂), 2.85 (6H.s,2xCH₃),1.55 (9H,s,3xCH₃).

### Intermediate 27. N,N-dimethyl-1-piperazinecarboxamide

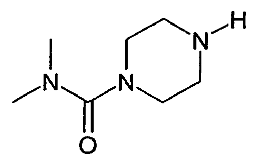

To intermediate 26 (0.27g) was added 4M hydrogen chloride in 1,4 dioxane (10ml); after stirring for 3h the solvent was evaporated *in vacuo* to give the title compound as a white solid (0.238g).
¹HNMR (MeOD) δ 3.5 (4H,m,CH₂), 3.3 (4H,m,2xCH₂), 2.95 (6H,s,2xCH₃), 3.35 (1H, m,NH)

### Intermediate 28. 4-Chloro-8-methyl-6-(4-morpholinylsulfonyl)-3-auinolinecarboxamide

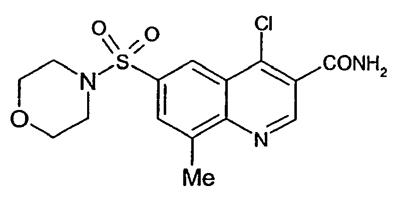

Chlorine was bubbled through a suspension of intermediate 10a (3.0g) in acetic acid (40ml) and water (10ml) for 4min. The mixture was partitioned between water (300ml) and diethyl ether (300ml) and the organic layer dried (MgSO₄) and concentrated *in vacuo.* The residue was triturated with toluene (75ml) and the solvent removed *in vacuo* to give a yellow solid (2.7g). The yellow solid (1g) in dichloromethane (3ml) and *N,N-*dimethylformamide (0.5ml) was added to a solution of morpholine (0.272g) and *N,N-*diisopropylethylamine (1ml) in dichloromethane (15ml) at 0°C. The mixture was stirred at 0°C for 2h and then allowed to warm to room temperature over 3h. The mixture was partitioned between saturated aqueous sodium bicarbonate solution (40ml) and dichloromethane (40ml). The organic layer was collected, dried (MgSO₄) and the solvent removed *in vacuo.* Purification by chromatography on silica gel, eluting with 30% cyclohexane in ethyl acetate gave the impure title compound as a fawn foam (0.6g). LC/MS Rt 2.52 min *m*/*z* 370 [MH⁺] ; major by product LC/MS Rt 2.70 min *m*/*z* 455 [MH⁺]

Similarly prepared from intermediate 10a and 1-acetylpiperazine (Aldrich) was the following:

### Intermediate 29. 6-[(4-Acetyl-1-piperazinyl)sulfonyl]-4-chloro-8-methyl-3-guinolinecarboxamide

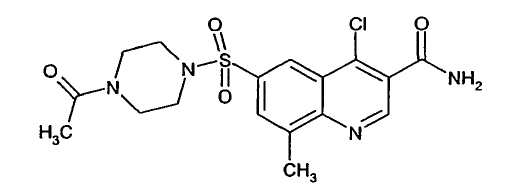

LC/MS Rt 2.41 min *m*/*z* 411 [MH⁺]

### Intermediate 30. 4-Chloro-6-({[2-(4-morpholinyl)ethyl]amino}sulfonyl)-3-quinolinecarboxamide

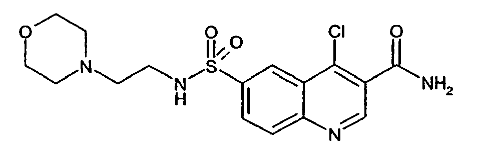

Chlorine was bubbled through a vigourously stirred suspension of intermediate 10 (2.5g) in acetic acid (40ml) and water (10ml) at room temperature for 10min. The mixture was partitioned between water (300ml) and diethyl ether (2x300ml) and the organic layers washed with brine, dried (MgSO₄) and concentrated *in vacuo.* The residue was triturated with toluene (2x150ml) and the solvent removed *in vacuo* to give the sulphonyl chloride intermediate as a yellow solid (3.3g). The yellow solid was dissolved in a mixture of dichloromethane (100ml) and *N,N*-dimethylformamide (11ml), and an aliquot (11.1ml) of this solution was added to a solution of [2-(4-morpholinyl)ethyl]amine (Aldrich, 0.136g) and *N,N*-diisopropylethylamine (0.367ml) in dichloromethane (2ml) at 0°C. The mixture was stirred at 0°C for 2h and then allowed to warm to room temperature overnight. The mixture was partitioned between saturated aqueous sodium bicarbonate solution (20ml) and dichloromethane (10ml). The organic layer was separated using a hydrophobic frit, and the solvent removed *in vacuo* to give the title compound as a brown gum (0.334g). LC/MS Rt 1.77 min m/z 399 [MH⁺]

Similarly prepared from intermediate 10 were the following:

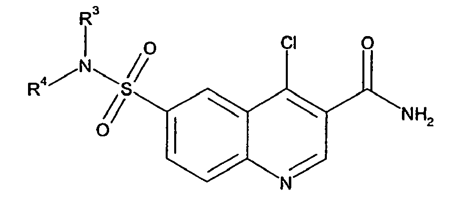

| **Intermediate Number** | **R³R⁴N**- | **Amine Reagent R³R⁴NH/ Source** | **LC/MS Rt min** | **LC/MS MH⁺** |
|---|---|---|---|---|
| 31 | 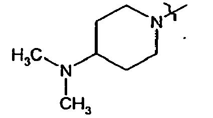 | N,N-dimethyl-4-piperidinamine/ Lancaster | 1.89 | 397 |
| 32 | 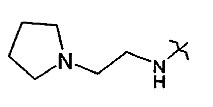 | [2-(1-pyrrolidinyl)ethyl] amine/ Aldrich | 1.79 | 383 |

### Intermediate 33. 4-Chloro-6-[(methylamino)sulfonyl]-3-quinolinecarboxamide

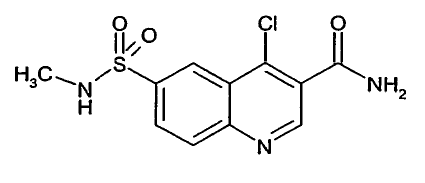

Chlorine was bubbled through a vigourously stirred suspension of intermediate 10 (0.1g) in acetic acid (4ml) and water (1ml) at room temperature for 2min. The mixture was partitioned between water (100ml) and diethyl ether (3x50ml) and the organic layers washed with brine, dried (MgSO₄) and concentrated *in vacuo.* The residue was triturated with toluene (2x15ml) and the solvent removed *in vacuo* to give the sulphonyl chloride intermediate as an orange gum (0.127g). The orange gum was dissolved in *N,N-*dimethylformamide (2.6ml), and an aliquot (1.3ml) of this solution was cooled to 0°C. Methylamine hydrochloride (Aldrich, 0.0074g) and *N,N*-diisopropylethylamine (0.058ml) were added, and the mixture was allowed to warm to room temperature with stirring overnight (18h). The mixture was applied directly to a solid phase extraction cartridge (Isolute, aminopropyl solid phase, 10g), and eluted sequentially with chloroform, ether, ethyl acetate, acetone and methanol. The acetone and methanol fractions were evaporated to give the title compound (0.0109g).
LC/MS Rt 2.04 min *m*/*z* 300 [MH⁺]

### Examples

### Example 1. -4-{[3-(Methyloxy)phenyl]amino}-6-(1-piperidinylsulfonyl)-3 quinolinecarboxamide hydrochloride

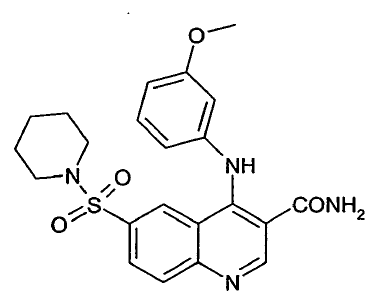

Intermediate 4 (0.011g) was suspended in acetonitrile (2ml), 3-methoxyaniline (0.004g) (available from Aldrich) was added and the mixture heated at 70°C for 5h. The mixture was cooled to room temperature, and the precipitate filtered off, washed with acetonitrile and dried to give the title compound (0.008g).
LC/MS Rt 2.75min *m*/*z* 441 [MH⁺]

Similarly prepared from the intermediate numbers shown in the table were the following:

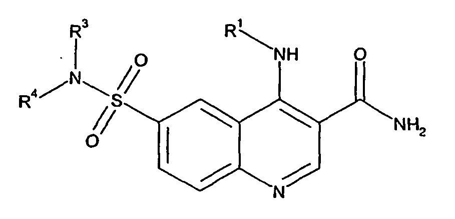

| **Example Number (a)** | **Intermediate number** | **R³ R⁴ N-** | **R¹** | **Amine Reagent R¹NH_{2/} Source** | **Isolation Method (b)** | **LC/MS MH⁺** | **LC/MS Rt min** |
|---|---|---|---|---|---|---|---|
| 2 (HCl) | 16 | 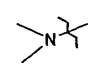 | 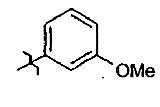 | 3-(methyloxy)aniline/ Aldrich | (I) | 401 | 2.41 |
| 3 | 12 | 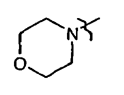 | 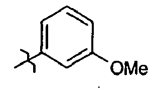 | 3-(methyloxy)aniline/ Aldrich | (II) | 443 | 2.44 |
| 4 (HCl) | 12 | 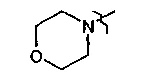 | 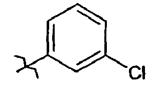 | 3-chloroaniline/ Aldrich | (II)* | 447 | 2.64 |
| 5 (HCl) | 12 | 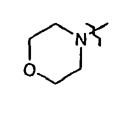 | 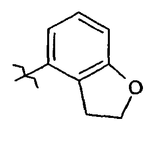 | 2,3-dihydro-1-benzofuran-4-amine hydrobromide/ Journal of Heterocyclic Chemistry (1980), 17(6), 1333-5. | (II)* | 455 | 2.41 |
| 6 (HCl) | 12 | 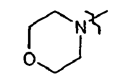 | 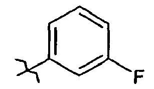 | 3-fluoroaniline/ Aldrich | (II)* | 431 | 2.51 |
| 7 (HCl) | 12 | 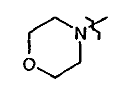 | 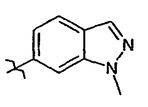 | 1-methyl-1*H*-indazol-6-amine hydrochloride/ Synthetic Communications (1996), 26(13), 2443-2447. | (I) | 467 | 2.29 |
| 8 (HCl) | 12 | 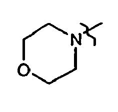 | 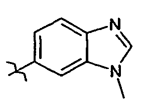 | 1-methyl-1*H*-benzimidazol-6-amine/ ,Heterocycles (1991) 32(5), 1003-12. | (I) | 467 | 1.90 |
| 9 (HCl) | 16 | 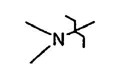 | 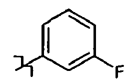 | 3-fluoroaniline/ Aldrich | (I) | 443 | 2.39 |
| 10 (HCl) | 13 | 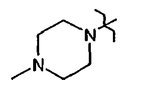 | 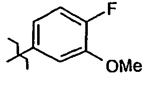 | 4-fluoro-3-methoxyaniline/ Apollo-Chem | (II)* | 473 | 2.03 |
| 11 (HCl) | 14 | 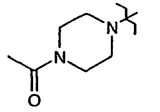 | 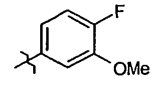 | 4-fluoro-3-methoxyaniline/ Apollo-Chem | (I) | 501 | 2.32 |
| 12 (HCl) | 15 | 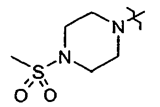 | 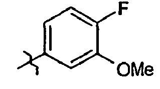 | 4-fluoro-3-methoxyaniline/ Apollo-Chem | (I) | 537 | 2.53 |
| 13 (HCl) | 14 | 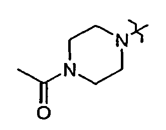 | 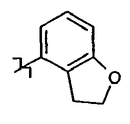 | 2,3-dihydro-1-benzofuran-4-amine hydrobromide/ Journal of Heterocyclic Chemistry (1980), 17(6), 1333-5. | (I) | 495 | 2.30 |
| 14 (HCl) | 15 | 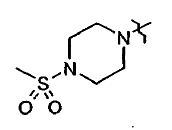 | 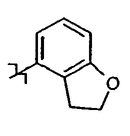 | 2,3-dihydro-1-benzofuran-4-amine hydrobromide/ Journal of Heterocyclic Chemistry (1980), 17(6), 1333-5. | (I) | 531 | 2.51 |
| 15 (HCl) | 12 | 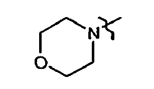 | 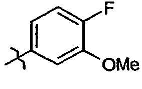 | 4-fluoro-3-methoxyaniline/ APOLLO-CHEM | (I) | 461 | 2.35 |
| 16 (HCl) | 16 | 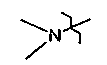 | 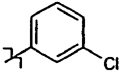 | 3-chloroaniline/ Aldrich | (I) | 405 | 2.64 |
| 17 (HCl) | 16 | 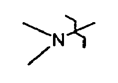 | 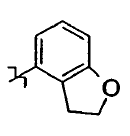 | 2,3-dihydro-1-benzofuran-4-amine hydrobromide/ Journal of Heterocyclic Chemistry (1980), 17(6), 1333-5. | (I) | 412 | 2.37 |
| 18 (HCI) | 17 | 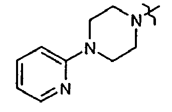 | 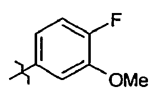 | 4-fluoro-3-methoxyaniline/ Apollo-Chem | (I) | 537 | 2.56 |
| 19 (HCl) | 18 | 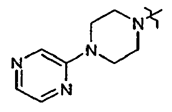 | 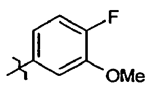 | 4-fluoro-3-methoxyaniline/ Apollo-Chem | (II)* | 538 | 2.66 |
| 20 (HCl) | 19 | 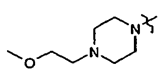 | 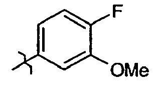 | 4-fluoro-3-methoxyaniline/ Apollo-Chem | (I) | 518 | 2.11 |
| 21 (HCl) | 20 | 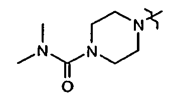 | 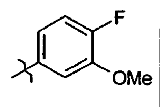 | 4-fluoro-3-methoxyaniline/ Apollo-Chem | (I) | 531 | 2.50 |
| 22 (HCI) | 18 | 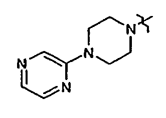 | 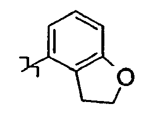 | 2,3-dihydro-1-benzofuran-4-amine hydrobromide/ Journal of Heterocyclic Chemistry (1980), 17(6), 1333-5. | (II)* | 532 | 2.64 |
| 23 (HCl) | 20 | 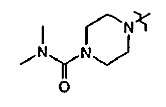 | 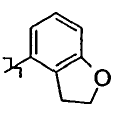 | 2,3-dihydro-1-benzofuran-4-amine hydrobromide/ Journal of Heterocyclic Chemistry (1980), 17(6), 1333-5. | (I) | 525 | 2.48 |
| 24 (TFA) | 21 | 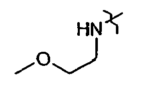 | 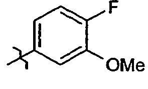 | 4-fluoro-3-methoxyaniline/ Apollo-Chem | (III) | 449 | 2.22 |
| 25 (TFA) | 21 | 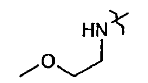 | 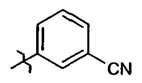 | 3-aminobenzonitrile/ Aldrich | (III) | 426 | 2.23 |
| 26 (TFA) | 21 | 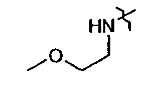 | 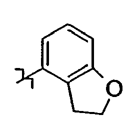 | 2,3-dihydro-1-benzofuran-4-amine hydrobromide/ Journal of Heterocyclic Chemistry (1980), 17(6), 1333-5. | (III) | 443 | 2.20 |
| 27 (HCl) | 24 | 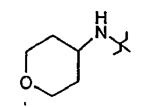 | 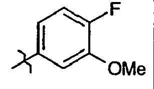 | 4-fluoro-3-methoxyaniline/ Apollo-Chem | (I) | 475 | 2.26 |
| 28 (TFA) | 24 | 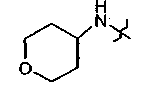 | 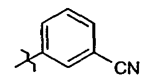 | 3-aminobenzonitrile/ . Aldrich | (III) | 452 | 2.27 |
| 29 (TFA) | 24 | 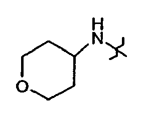 | 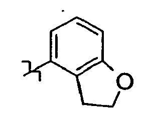 | 2,3-dihydro-1-benzofuran-4-amine hydrobromide/ Journal of Heterocyclic Chemistry (1980), 17(6), 1333-5 | (III) | 469 | 2.24 |
| 30 (HCl) | 24 | 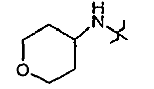 | 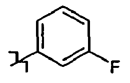 | 3-fluoroaniline/ Aldrich | (I) | 445 | 2.31 |
| 31 (TFA) | 22 | 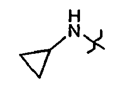 | 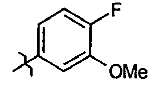 | 4-fluoro-3-methoxyaniline/ Apollo-Chem | (III) | 431 | 2.35 |
| 32 (TFA) | 22 | 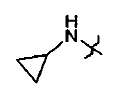 | 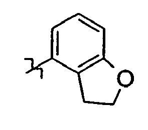 | 2,3-dihydro-1-benzofuran-4-amine hydrobromide/ Journal of Heterocyclic Chemistry (1980), 17(6), 1333-5 | (III) | 425 | 2.32 |
| 33 (TFA) | 22 | 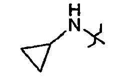 | 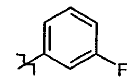 | 3-fluoroaniline/ Aldrich | (III) | 401 | 2.43 |
| 34 (HCI) | 23 | 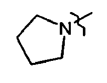 | 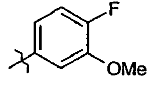 | 4-fluoro-3-methoxyaniline/ Apollo-Chem | (1) | 445 | 2.51 |
| 35 (HCl) | 23 | 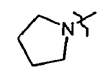 | 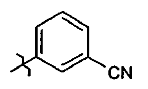 | 3-aminobenzonitrile/ Aldrich | (I) | 422 | 2.57 |
| 36 (HCl) | 23 | 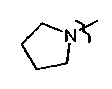 | 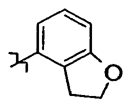 | 2,3-dihydro-1-benzofuran-4-amine hydrobromide/ Journal of Heterocyclic Chemistry (1980), 17(6), 1333-5 | (I) | 439 | 2.49 |
| 37 (HCI) | 23 | 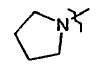 | 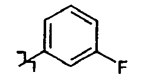 | 3-fluoroaniline/ Aldrich | (I) | 415 | 2.62 |
| 38 (HCl) | 16 | 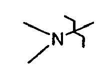 | 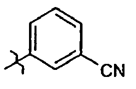 | 3-aminobenzonitrile/ Aldrich | (I) | 396 | 2.42 |
| 39 (TFA) | 21 | 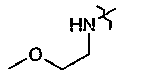 | 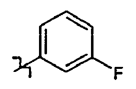 | 3-fluoroaniline/ Aldrich | (III) | 419 | 2.26 |
| 40 (TFA) | 22 | 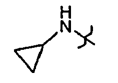 | 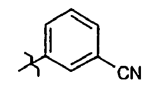 | 3-aminobenzonitrile/ Aldrich | (III) | 408 | 2.39 |
| 41 (TFA) | 16 | 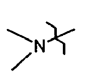 | 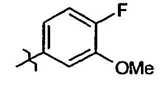 | 4-fluoro-3-methoxyaniline/ Apollo-Chem | (III) | 419 | 2.37 |

| | | | | | | | |
|---|---|---|---|---|---|---|---|
| (a) Salt forms: HCl = hydrochloride TFA = trifluoroacetate (b) Isolation Method: (I) Filtered off directly from the reaction mixture; it is thought that compounds isolated by this method are hydrochloride salts. (II) Mass Directed HPLC Method A; it is thought that compounds isolated by this method are free bases unless the R¹ or R³ groups contain basic moieties, in which case formate salts may be formed. *2M aqueous hydrochloric acid was added to the product fractions to form the HCl salt where indicated. (III) Mass Directed HPLC Method B; it is thought that compounds isolated by this method are trifluoroacetate salts. | | | | | | | |

### Example 42. 4-{[4-Fluoro-3-(methyloxy)phenyl]amino}-8-methyl-6-(4-morpholinylsulfonyl)-3-quinolinecarboxamide

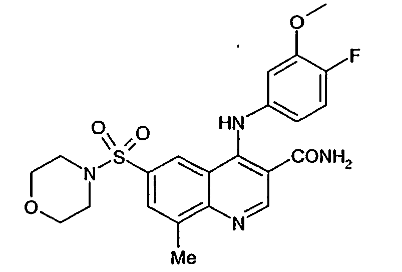

### Intermediate 28 (0.050g) was suspended in acetonitrile (2ml),

4-fluoro-3-(methyloxy)aniline (0.025g) (available from Aldrich) was added and the mixture heated at 80°C for 16h. The mixture was cooled to room temperature and the solvent blown off under a stream of nitrogen. Purification by mass directed HPLC gave a yellow oil. This was loaded onto an SPE cartridge (1g Varian Bond Elut, aminopropyl solid phase) and eluted with methanol to give the title compound as a yellow solid (0.018g). LC/MS Rt 2.58min *m*/*z* 475 [MH⁺]

Similarly prepared from the intermediate numbers shown in the table were the following:

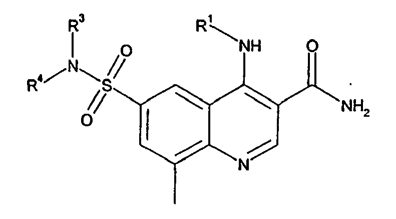

| **Example Number (a)** | **Intermediate number** | **R³R⁴N-** | **R¹** | **Amine Reagent R¹NH₂/ Source** | **Isolation Method (b)** | **LC/MS** LC/MS **Rt min** | **LC/MS** LC/MS **MH⁺** |
|---|---|---|---|---|---|---|---|
| 43 | 28 | 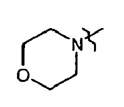 | 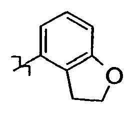 | 2,3-dihydro-1-benzofuran-4-amine hydrobromide/ Journal of Heterocyclic Chemistry (1980), 17(6), 1333-5 | (1) | 2.57 | 469 |
| 44 | 28 | 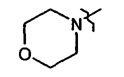 | 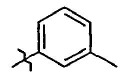 | 3-methylanilinel Aldrich | (1) | 2.56 | 441 |
| 45 | 28 | 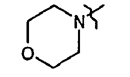 | 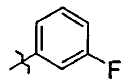 | 3-fluoroanilinel Aldrich | (1) | 2.68 | 445 |
| 46 | 28 | 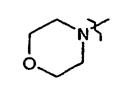 | 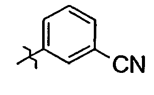 | 3-cyanoaniline/ Aldrich | (1) | 452 | 2.62 |
| 50 HCl | 28 | 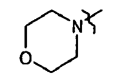 | 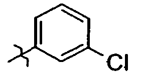 | 3-chloroaniline/ Aldrich | (II) | 461 | 2.83 |
| 51 HCl | 28 | 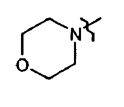 | 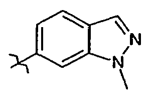 | 1-methyl-1*H*-indazol-6-amine hydrochloride/ Synthetic Communications (1996), 26(13), 2443-2447. | (II) | 481 | 2.49 |
| 52 | 29 | 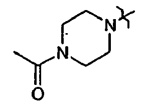 | 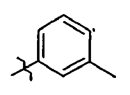 | 3-methylaniline/ Aldrich | (I) | 482 | 2.44 |
| 53 | 29 | 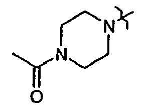 | 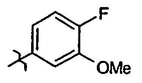 | 4-fluoro-3-methoxyaniline/ Apollo-Chem | (I) | 516 | 2.44 |
| 54 | 29 | 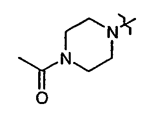 | 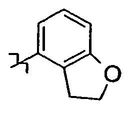 | 2,3-dihydro-1-benzofuran-4-amine hydrobromide/ Journal of Heterocyclic Chemistry (1980), 17(6), 1333-5 | (I) | 510 | 2.44 |

| | | | | | | | |
|---|---|---|---|---|---|---|---|
| (a) Salt form: HCl = hydrochloride (b) Isolation Method (1): Mass Directed HPLC Method A, followed by aminopropyl SPE to give the product as the free base. (II) Mass Directed HPLC Method A; hydrochloric acid was added to the product fractions to generate the hydrochloride salt | | | | | | | |

### Example 47. 4-{[4-Fluoro-3-(methyloxy)phenyl]amino}-6-({[2-(4-morpholinyl)ethyl]amino}sulfonyl)-3-quinolinecarboxamide hydrochloride

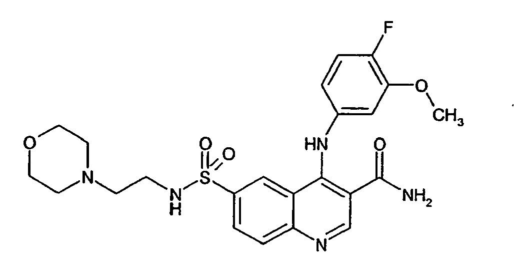

4-Fluoro-3-methoxyaniline (Apollo-Chem, 0.0085g) was added to intermediate 30 (0.021 g) in ethanol (3ml), pyridine hydrochloride 0.012g) was added, and the mixture was heated under reflux for 5h. The solvent was evaporated to give a brown gum (0.033g), which was purified by mass directed preparative HPLC (Method A); 2N hydrochloric acid (1ml) was added to the product fractions, and the solvents evaporated to give the title compound (0.0067g).
LC/MS Rt 1.91 min *m*/*z* 504 [MH⁺]

### Example 48. 4-(2,3-Dihydro-1-benzofuran-4-ylamino)-6-{[4-(dimethylamino)-1-piperidinyl]sulfonyl}-3-quinolinecarboxamide hydrochloride

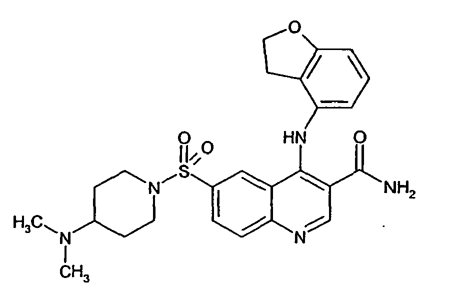

To intermediate 31 (0.042g) in ethanol (3ml) was added 2,3-dihydro-1-benzofuran-4-amine (Journal of Heterocyclic Chemistry (1980), 17(6), 1333-5, 0.016g) and concentrated hydrochloric acid (0.1ml), and the mixture was heated under reflux for 3h. The solvent was evaporated to give the crude product (0.070g), which was purified by mass directed HPLC (Method A); 2N hydrochloric acid (0.5ml) was added to the product fractions, and the solvents evaporated to give the title compound (0.0041g).
LC/MS Rt 1.89min m/z 496 [MH⁺]

Similarly prepared from intermediate 32 and 4-fluoro-3-methoxyaniline (Apollo-Chem) (except that no hydrochloric acid was added to the preparative HPLC fractions) was:

### Example 49. 4-{[4-Fluoro-3-(methyloxy)phenyl]amino}-6-({[2-(1-pyrrolidinyl)ethyl]amino}sulfonyl)-3-quinolinecarboxamide formate

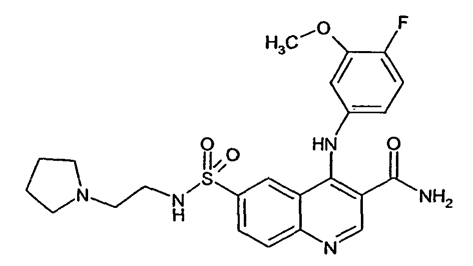

LC/MS Rt 1.79min *m*/*z* 488 [MH⁺]

### Example 55. 4-{[4-Fluoro-3-(methyloxy)phenyl]amino}-6-[(methylamino)sulfonyl]-3-quinolinecarboxamide hydrochloride

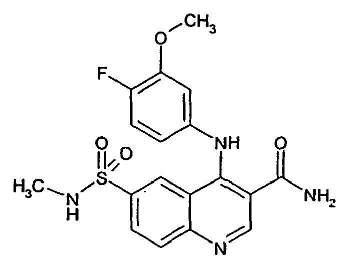

To a solution of intermediate 33 (0.011g) in acetonitrile (2ml) and N,N-dimethylformamide (1.5ml) was added 4-fluoro-3-methoxyaniline (Apollo-Chem, 0.007g), and the mixture was heated at 80° with stirring under nitrogen for 18h. The solvents were evaporated to give a brown gum, which was purified by mass directed preparative HPLC (Method A); 2N hydrochloric acid (0.5ml) was added to the product fractions, and the solvents evaporated to give the title compound as a pale yellow solid (0.0058g).
LC/MS Rt 2.17min m/z 405 [MH⁺]

## Claims

1. A compound of formula (I) or a pharmaceutically acceptable salt thereof: 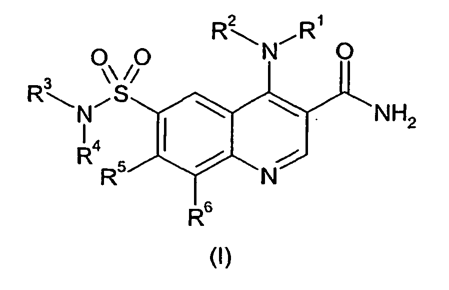 wherein:
R¹ is
Aryl optionally substituted by one or more substituents selected from C₁₋₆alkyl, C₁₋₆alkoxy, halogen, C₁₋₆alkylCO-, -(CH₂)ₘOH, -CN, R⁷R⁸N-;
Aryl fused to a C₄₋₇cycloalkyl ring;
Aryl fused to a heterocyclyl ring;
Heteroaryl wherein the heteroaryl is optionally substituted by one or more substituents selected from: C₁₋₆alkyl, N-oxide, C₁₋₆alkoxy;
Heterocyclyl.
R² is hydrogen or C₁₋₆alkyl;
R³ is
Hydrogen;
C₁₋₆alkyl optionally substituted by one or more substituents selected from: heterocyclyl (itself optionally substituted by C₁₋₆alkyl), R⁹R¹⁰NCO-, R¹¹CONR¹²-, C₁₋₆alkylSO₂NR¹³-, C₁₋₆alkoxy, R¹⁴R¹⁵N-;
C₃₋₇cycloalkyl;
Aryl or aryl(C₁₋₆alkyl) wherein the aryl is optionally substituted by one or more substituents selected from: C₁₋₆alkyl, C₁₋₆alkoxy, halogen, R¹⁶R¹⁷NCO-;
Aryl fused to C₄₋₇cycloalkyl, wherein the cycloalkyl is optionally substituted by =O;
Heteroaryl or heteroaryl(C₁₋₆alkyl), wherein the heteroaryl is optionally substituted by one or more substituents selected from C₁₋₆alkyl, C₁₋₆alkoxy, halogen;
Heterocyclyl optionally substituted by one or more C₁₋₆alkyl, C₁₋₆alkylCO-, C₁₋₆alkylSO₂-, R¹⁸R¹⁹NCO-, C₁₋₆alkoxyCO-;
R⁴ is hydrogen or C₁₋₆alkyl;
R³ and R⁴ together with the nitrogen atom to which they are attached may form a heterocyclyl ring, which is optionally substituted by one or more substituents selected from C₁₋₆alkyl (optionally substituted by one or more OH or C₁₋₆alkoxy groups), C₁₋₆alkoxy, C₁₋₆alkoxyCO-, C₃₋₇cycloalkyl (optionally substituted by OH), C₁₋₆alkylCO-, C₁₋₆alkylSO₂-, OH, -(CH₂)ₘNR²⁰R²¹, -(CH₂)ₘCONR²²R²³, -(CH₂)ₘNR²⁴COR²⁵, C₁₋₆alkoxyC₁₋₄alkyl, aryICO-heteroaryl, heteroarylC₁₋₄alkyl, heteroarylCO.
m is 0-6
R⁵ is hydrogen or C₁₋₆alkyl;
R⁶ is hydrogen, C₁₋₆alkyl, C₁₋₆alkoxy, fluorine, chlorine, or bromine;
R⁷⁻²⁵ all independently represent hydrogen, C₁₋₆ alkyl;
R¹⁴ and R¹⁵ together with the nitrogen atom to which they are attached may form a heterocyclyl ring;
R¹⁶ and R¹⁷ together with the nitrogen atom to which they are attached may form a heterocyclyl ring;
R¹⁸ and R¹⁹ together with the nitrogen atom to which they are attached may form a heterocyclyl ring;
R²⁰ and R²¹ together with the nitrogen atom to which they are attached may form a heterocyclyl ring;
R²² and R²³ together with the nitrogen atom to which they are attached may form a heterocyclyl ring wherein:
- aryl is a mono-or bicyclic carbocyclic aromatic ring system containing up to 10 carbon atoms in the ring system
- heteroaryl is a monocyclic five-to seven-membered heterocyclic aromatic ring containing one or more heteroatoms selected from oxygen, nitrogen and sulfur or a fused bicyclic heterocyclic aromatic ring systems containing at least one heteroatom selected from oxygen, nitrogen and sulfur
- heterocyclyl is a monocyclic three-to seven-membered saturated or non-aromatic, unsaturated ring containing at least one heteroatom selected from oxygen, nitrogen and sulfur.

2. A compound according to claim 1 wherein R¹ is selected from
aryl optionally substituted by one or more substituents selected from C₁₋₆alkyl, C₁₋₆alkoxy-, halogen, -CN;
aryl fused to a heterocyclyl ring;
heteroaryl optionally substituted by one or more substituents selected from: C₁₋₆alkyl.

3. A compound according to claim 1 or 2 wherein R² is hydrogen.

4. A compound according to any of claims 1 to 3 wherein R³ is selected from
C₁₋₆alkyl optionally substituted by one or more substituents selected from heterocyclyl, C₁₋₆alkoxy;
C₃₋₇cycloalkyl;
Heterocyclyl.

5. A compound according to any of claims 1 to 4 wherein R⁴ is hydrogen or C₁₋₆alkyl.

6. A compound according to any of claims 1 to 3 wherein R³ and R⁴ together with the nitrogen atom to which they are attached may form a heterocyclyl ring, optionally substituted by one or more substituents selected from C₁₋₆alkyl (optionally substituted by one or more C₁₋₆alkoxy groups), C₁₋₆alkyICO, C₁₋₆alkylSO₂; -(CH₂)ₘCONR²²R²³, - (CH₂)ₘNR²⁰R²¹, heteroaryl.

7. A compound according to any of claims 1 to 6 wherein R⁵ is hydrogen.

8. A compound according to any of claims 1 to 7 wherein R⁶ is hydrogen or C₁₋₆alkyl.

9. A compound according to claim 1 wherein
R¹ is selected from
phenyl optionally substituted by one or more substituents selected from methyl, methoxy, fluoro, chloro, cyano;
dihydrobenzofuranyl;
indazolyl or benzimidazolyl optionally substituted by methyl;
R² is hydrogen;
R³ is selected from
C₁₋₃alkyl optionally substituted by one C₁₋₂alkoxy group or a 5 to 7 membered saturated ring containing one or two heteratoms selected from nitrogen or oxygen;
C₃₋₅cycloalkyl;
5 to 7 membered saturated ring containing one heteroatom which is oxygen;
R⁴ is hydrogen or C₁₋₆alkyl;
R⁵ is hydrogen;
R⁶ is hydrogen or C₁₋₆alkyl.

10. A compound according to claim 1 wherein
R¹ is selected from
phenyl optionally substituted by one or more substituents selected from methyl, methoxy, fluoro, chloro, cyano;
dihydrobenzofuranyl;
indazolyl or benzimidazolyl optionally substituted by methyl;
R² is hydrogen;
R³ and R⁴ together with the nitrogen atom to which they are attached may form a 5 or 6 membered heterocyclyl ring, optionally substituted by one or more substituents selected from C₁₋₃alkyl (optionally substituted by one or more C₁₋₂alkoxy groups), C₁₋₃alkylCO, C₁₋₃alky[SO₂; -CON(CH₃)₂, -N(CH₃)₂, pyrazinyl, pyridinyl;
R⁵ is hydrogen;
R⁶ is hydrogen or C₁₋₆alkyl.

11. A compound of formula (I) selected from the group consisting of
6-[(dimethylamino)sulfonyl]-4-{[3-(methyloxy)phenyl]amino}-3-quinolinecarboxamide ; 4-(2,3-dihydro-1-benzofuran-4-ylamino)-6-(4-morpholinylsulfonyl)-3-quinolinecarboxamide;
6-[(4-acetyl-1-piperazinyl)sulfonyl]-4-{[4-fluoro-3-(methyloxy)phenyl]amino}-3-quinolinecarboxamide;
4-{[4-fluoro-3-(methyloxy)phenyl]amino}-6-{[4-(methylsulfonyl)-1-piperazinyl]sulfonyl}-3-quinolinecarboxamide;
6-[(4-acetyl-1-piperazinyl)sulfonyl]-4-(2,3-dihydro-1-benzofuran-4-ylamino)-3-quinolinecarboxamide;
4-(2,3-dihydro-1-benzofuran-4-ylamino)-6-{[4-(methylsulfonyl)-1-piperazinyl]sulfonyl}-3-quinolinecarboxamide;
4-(2,3-dihydro-1-benzofuran-4-ylamino)-6-[(dimethylamino)sulfonyl]-3-quinolinecarboxamide;
6-({4-[(dimethylamino)carbonyl]-1-piperazinyl}sulfonyl)-4-{[4-fluoro-3-(methyloxy)phenyl]amino}-3-quinolinecarboxamide;
4-(2,3-dihydro-1-benzofuran-4-ylamino)-6-{[4-(2-pyrazinyl)-1-piperazinyl]sulfonyl}-3-quinolinecarboxamide;
4-(2,3-dihydro-1-benzofuran-4-ylamino)-6-({4-[(dimethylamino)carbonyl]-1-piperazinyl}sulfonyl)-3-quinolinecarboxamide;
4-(2,3-dihydro-1-benzofuran-4-ylamino)-6-[(tetrahydro-2*H*-pyran-4-ylamino)sulfonyl]-3-quinolinecarboxamide;
4-{[4-fluoro-3-(methyloxy)phenyl]amino}-8-methyl-6-(4-morpholinylsulfonyl)-3-quinolinecarboxamide
4-(2,3-dihydro-1-benzofuran-4-ylamino)-8-methyl-6-(4-morpholinylsulfonyl)-3-quinolinecarboxamide
8-methyl-4-[(3-methylphenyl)amino]-6-(4-morpholinylsulfonyl)-3-quinolinecarboxamide
4-[(3-fluorophenyl)amino]-8-methyl-6-(4-morpholinylsulfonyl)-3-quinolinecarboxamide
4-[(3-cyanophenyl)amino]-8-methyl-6-(4-morpholinylsulfonyl)-3-quinolinecarboxamide
4-(2,3-dihydro-1-benzofuran-4-ylamino)-6-{[4-(dimethylamino)-1-piperidinyl]sulfonyl}-3-quinolinecarboxamide
4-[(3-chlorophenyl)amino]-8-methyl-6-(4-morpholinytsulfonyl)-3-quinolinecarboxamide
8-methyl-4-[(1-methyl-1 H-indazol-6-yl)amino]-6-(4-morpholinylsulfonyl)-3-quinolinecarboxamide
6-[(4-acetyl-1-piperazinyl)sulfonyl]-8-methyl-4-[(3-methylphenyl)amino]-3-quinolinecarboxamide
6-[(4-acetyl-1-piperazinyl)sulfonyl]-4-{[4-fluoro-3-(methyloxy)phenyl]amino}-8-methyl-3-quinolinecarboxamide
6-[(4-acetyl-1-piperazinyl)sulfonyl]-4-(2,3-dihydro-1-benzofuran-4-ylamino)-8-methyl-3-quinolinecarboxamide
and pharmaceutically acceptable salts thereof.

12. A process for the preparation of a compound of formula (I) and pharmaceutically acceptable salts thereof as defined in any of claims 1 to 11 which comprises:
(A) reacting a compound of formula (II); 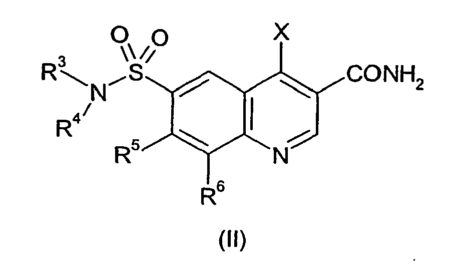 wherein R³, R⁴, R⁵ and R⁶ are as defined above, and X represents a halogen atom, with an amine of formula R¹R²NH, wherein R¹ and R² are as defined above; or
(B) interconversion of a compound of formula (I) into another compound of formula (I); or
(C) deprotecting a protected derivative of a compound of formula (I).

13. A compound or a pharmaceutically acceptable salt thereof, according to any of claims 1 to 11, for use in therapy.

14. The use of a compound according to any of claims 1 to 11, or a pharmaceutically acceptable salt thereof, in the manufacture of a medicament for the treatment or prophylaxis of inflammatory and/or allergic diseases.

15. A pharmaceutical composition which comprises a compound according to any of claims 1 to 11, or a pharmaceutically acceptable salt thereof optionally with a pharmaceutically acceptable carrier or excipient.

16. A pharmaceutical composition according to claim 16 which is suitable for inhaled administration.

17. A pharmaceutical composition according to claim 16 which is suitable for oral administration.

18. A pharmaceutical composition according to claim 16 which is suitable for topical administration.

## Patentansprüche

1. Verbindung gemäß Formel (I) oder ein pharmazeutisch annehmbares Salz davon: 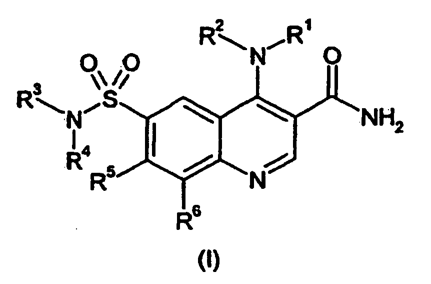 worin:
R¹ Aryl ist, optional substituiert durch einen oder mehr Substituenten, gewählt aus C₁₋₆-Alkyl, C₁₋₆-Alkoxy, Halogen, C₁₋₆-Alkyl-CO-, -(CH₂)ₘOH, -CN, R⁷R⁸N-;
Aryl, fusioniert an einen C₄₋₇-Alkyl-Ring;
Aryl, fusioniert an einen Heterocyclyl-Ring;
Heteroaryl, wobei das Heteroaryl optional durch ein oder mehr Substituenten substituiert ist, gewählt aus: C₁₋₆-Alkyl, N-Oxid, C₁₋₆-Alkoxy;
Heterocyclyl;
R² ist Wasserstoff oder C₁₋₆-Alkyl ;
R³ ist Wasserstoff;
C₁₋₆-Alkyl, optional substituiert durch ein oder mehr Substituenten, gewählt aus: Heterocyclyl (selbst optional substituiert durch C₁₋₆-Alkyl), R⁹R¹⁰NCO-, R¹¹CONR¹²-, C₁₋₆-Alkyl-SO₂NR¹³-, C₁₋₆-Alkoxy, R¹⁴R¹⁵N-;
C₃₋₇-Cycloalkyl;
Aryl oder Aryl(C₁₋₆-alkyl), wobei das Aryl optional durch ein oder mehr Substituenten substituiert ist, gewählt aus: C₁₋₆-Alkyl, C₁₋₆-Alkoxy, Halogen, R¹⁶R¹⁷NCO-;
Aryl, fusioniert an C₄₋₇-Cycloalkyl, wobei das Cycloalkyl optional durch =O substituiert ist;
Heteroaryl oder Heteroaryl(C₁₋₆-Alkyl), wobei das Heteroaryl optional substituiert ist durch ein oder mehr Substituenten, gewählt aus C₁₋₆-Alkyl, C₁₋₆-Alkoxy, Halogen;
Heterocyclyl, optional substituiert durch ein oder mehr C₁₋₆-Alkyl, C₁₋₆-Alkyl-CO-, C₁₋₆-Alkyl-SO₂-, R¹⁸R¹⁹NCO-, C₁₋₆-Alkoxy-CO-;
R⁴ ist Wasserstoff oder C₁₋₆-Alkyl;
R³ und R⁴ können zusammen mit dem Stickstoffatom, an das sie angebunden sind, einen Heterocyclyl-Ring bilden, der optional durch ein oder mehr Substituenten substituiert ist, gewählt aus C₁₋₆-Alkyl (optional substituiert durch ein oder mehr OH- oder C₁₋₆-AlkoxyGruppen), C₁₋₆-Alkoxy, C₁₋₆-Alkoxy-CO-, C₃₋₇-Cycloalkyl (optional substituiert durch OH), C₁₋₆-Alkyl-CO-, C₁₋₆-Alkyl-SO₂-, OH, -(CH₂)ₘNR²⁰R²¹, -(CH₂)ₘCONR²²R²³, -(CH₂)ₘNR²⁴COR²⁵, C₁₋₆-Alkoxy-C₁₋₄-alkyl, Aryl-CO-Heteroaryl, Heteroaryl-C₁₋₄-alkyl, Heteroaryl-CO;
m ist 0-6;
R⁵ ist Wasserstoff oder C₁₋₆-Alkyl;
R⁶ ist Wasserstoff, C₁₋₆-Alkyl, C₁₋₆-Alkoxy, Fluor, Chlor oder Brom;
R⁷⁻²⁵ bedeuteten alle unabhängig Wasserstoff, C₁₋₆-Alkyl;
R¹⁴ und R¹⁵ können zusammen mit dem Stickstoffatom, an das sie angebunden sind, einen Heterocyclyl-Ring bilden;
R¹⁶ und R¹⁷ können zusammen mit dem Stickstoffatom, an das sie angebunden sind, einen Heterocyclyl-Ring bilden;
R¹⁸ und R¹⁹ können zusammen mit dem Stickstoffatom, an das sie angebunden sind, einen Heterocyclyl-Ring bilden;
R²⁰ und R²¹ können zusammen mit dem Stickstoffatom, an das sie angebunden sind, einen Heterocyclyl-Ring bilden;
R²² und R²³ können zusammen mit dem Stickstoffatom, an das sie angebunden sind, einen Heterocyclyl-Ring bilden;
worin
-Aryl ein mono- oder bicyclisches carbocyclisches aromatisches Ringsystem ist, enthaltend bis zu 10 Kohlenstoffatome im Ringsystem;
-Heteroaryl ein monocyclischer 5- bis 7-gliedriger heterocyclischer aromatischer Ring ist, enthaltend ein oder mehr Heteroatome, gewählt aus Sauerstoff, Stickstoff und Schwefel oder ein fusioniertes bicyclisches heterocyclisches aromatisches Ringsystem, enthaltend mindestens ein Heteroatom, gewählt aus Sauerstoff, Stickstoff und Schwefel;
-Heterocyclyl ein monocyclischer 3- bis 7-gliedriger gesättigter oder nicht-aromatischer, ungesättigter Ring ist, enthaltend mindestens ein Heteroatom, gewählt aus Sauerstoff, Stickstoff und Schwefel.

2. Verbindung gemäß Anspruch 1, wobei R¹ gewählt ist aus
Aryl, optional substituiert durch ein oder mehr Substituenten, gewählt aus C₁₋₆-Alkyl, C₁₋₆-Alkoxy-, Halogen, -CN;
Aryl, fusioniert an einen Heterocyclyl-Ring;
Heteroaryl, optional substituiert durch ein oder mehr Substituenten, gewählt aus: C₁₋₆-Alkyl.

3. Verbindung gemäß Anspruch 1 oder 2, worin R² Wasserstoff ist.

4. Verbindung gemäß einem der Ansprüche 1 bis 3, worin R³ gewählt ist aus
C₁₋₆-Alkyl, optional substituiert durch einen oder mehr Substituenten, gewählt aus Heterocyclyl, C₁₋₆-Alkoxy;
C₃₋₇-Cycloalkyl;
Heterocyclyl.

5. Verbindung gemäß einem der Ansprüche 1 bis 4, worin R⁴ Wasserstoff oder C₁₋₆-Alkyl ist.

6. Verbindung gemäß einem der Ansprüche 1 bis 3, worin R³ und R⁴ zusammen mit dem Stickstoffatom, an das sie angebunden sind, einen Heterocyclyl-Ring bilden können, optional substituiert durch einen oder mehr Substituenten gewählt aus C₁₋₆-Alkyl (optional substituiert durch ein oder mehr C₁₋₆-Alkoxy-Gruppen), C₁₋₆-Alkyl-CO, C₁₋₆-Alkyl-SO₂; -(CH₂)ₘCONR²²R²³, -(CH₂)ₘNR²⁰R²¹, Heteroaryl.

7. Verbindung gemäß einem der Ansprüche 1 bis 6, worin R⁵ Wasserstoff ist.

8. Verbindung gemäß einem der Ansprüche 1 bis 7, worin R⁶ Wasserstoff oder C₁₋₆-Alkyl ist.

9. Verbindung gemäß Anspruch 1, worin
R¹ gewählt ist aus
Phenyl, optional substituiert durch ein oder mehr Substituenten, gewählt aus Methyl, Methoxy, Fluor, Chlor, Cyano;
Dihydrobenzofuranyl;
Indazolyl oder Benzimidazolyl optional substituiert durch Methyl;
R² ist Wasserstoff;
R³ ist gewählt aus
C₁₋₃-Alkyl, optional substituiert durch eine C₁₋₂-Alkoxy-Gruppe oder einen 5- bis 7-gliedrigen gesättigten Ring, enthaltend 1 oder 2 Heteroatome, gewählt aus Stickstoff oder Sauerstoff;
C₃₋₅-Cycloalkyl;
einem 5- bis 7-gliedrigen gesättigten Ring, enthaltend ein Heteroatom, das Sauerstoff ist;
R⁴ ist Wasserstoff oder C₁₋₆-Alkyl;
R⁵ ist Wasserstoff;
R⁶ ist Wasserstoff oder C₁₋₆-Alkyl.

10. Verbindung gemäß Anspruch 1 worin
R¹ gewählt ist aus
Phenyl, optional substituiert durch einen oder mehr Substituenten, gewählt aus Methyl, Methoxy, Fluor, Chlor, Cyano;
Dihydrobenzofuranyl;
Indazolyl oder Benzimidazolyl optional substituiert durch Methyl;
R² ist Wasserstoff;
R³ und R⁴ können zusammen mit dem Stickstoffatom, an das sie angebunden sind, einen 5- oder 6-gliedrigen Heterocyclyl-Ring bilden, optional substituiert durch ein oder mehr Substituenten, gewählt aus C₁₋₃-Alkyl (optional substituiert durch ein oder mehr C₁₋₂-Alkoxy-Gruppen), C₁₋₃-Alkyl-CO, C₁₋₃-Alkyl-SO₂; -CON(CH₃)₂, -N(CH₃)₂, Pyrazinyl, Pyridinyl;
R⁵ ist Wasserstoff;
R⁶ ist Wasserstoff oder C₁₋₆-Alkyl.

11. Verbindung gemäß Formel (I), gewählt aus der Gruppe bestehend aus
6-[(Dimethylamino)sulfonyl]-4-{[3-(methyloxy)phenyl]amino}-3-chinolincarboxamid ;
4-(2,3-Dihydro-1-benzofuran-4-ylamino)-6-(4-morpholinylsulfonyl)-3-chinolincarboxamid;
6-[(4-Acetyl-1-piperazinyl)sulfonyl]-4-{[4-fluor-3-(methyloxy)phenyl]amino}-3-chinolincarboxamid ;
4-{[4-Fluor-3-(methyloxy)phenyl]amino}-6-{[4-(methylsulfonyl)-1-piperazinyl]sulfonyl}-3-chinolincarboxamid;
6-[(4-Acetyl-1-piperazinyl)sulfonyl]-4-(2,3-dihydro-1-benzofuran-4-ylamino)-3-chinolincarboxamid;
4-(2,3-Dihydro-1-benzofuran-4-ylamino)-6-{[4-(methylsulfonyl)-1-piperazinyl]sulfonyl-3-chinolincarboxamid;
4-(2,3-Dihydro-1-benzofuran-4-ylamino)-6-[(dimethylamino)sulfonyl-3-chinolincarboxamid;
6-({4-[(Dimethylamino)carbonyl]-1-piperazinyl}-sulfonyl)-4-{[4-fluor-3-(methyloxy)phenyl]amino}-3-chinolincarboxamid;
4-(2,3-Dihydro-1-benzofuran-4-ylamino)-6-{[4-(2-pyrazinyl)-1-piperazinyl]sulfonyl}-3-chinolincarboxamid;
4-(2,3-Dihydro-l-benzofuran-4-ylamino)-6-({4-[(dimethylamino)carbonyl]-1-piperazinyl}sulfonyl)-3-chinolincarboxamid;
4-(2,3-Dihydro-1-benzofuran-4-ylamino)-6-[(tetrahydro-2H-pyran-4-ylamino)sulfonyl]-3-chinolincarboxamid;
4-([4-Fluor-3-(methyloxy)phenyl]amino)-8-methyl-6-(4-morpholinylsulfonyl)-3-chinolincarboxamid;
4-(2,3-Dihydro-l-benzofuran-4-ylamino)-8-methyl-6-(4-morpholinylsulfonyl)-3-chinolincarboxamid;
8-Methyl-4-[(3-methylphenyl)amino]-6-(4-morpholinylsulfonyl)-3-chinolincarboxamid,
4-[(3-Fluorphenyl)amino]-8-methyl-6-(4-morpholinylsulfonyl)-3-chinolincarboxamid;
4-[(3-Cyanophenyl)amino]-8-methyl-6-(4-morpholinylsulfonyl)-3-chinolincarboxamid;
4-(2,3-Dihydro-1-benzofuran-4-ylamino)-6-{[4-(dimethylamino)-1-piperidinyl]sulfonyl}-3-chinolincarboxamid;
4-[(3-Chlorphenyl)amino]-8-methyl-6-(4-morpholinylsulfonyl)-3-chinolincarboxamid;
8-Methyl-4-[(1-methyl-1H-indazol-6-yl)amino]-6-(4-morpholinylsulfonyl)-3-chinolincarboxamid;
6-[(4-Acetyl-1-piperazinyl)sulfonyl]-8-methyl-4-[(3-methylphenyl)amino]-3-chinolincarboxamid;
6-[(4-Acetyl-1-piperazinyl)sulfonyl]-4-{[4-fluor-3-(methyloxy)phenyl]amino}-8-methyl-3-chinolincarboxamid;
6-[(4-Acetyl-1-piperazinyl)sulfonyl]-4-(2,3-dihydro-1-benzofuran-4-ylamino)-8-methyl-3-chinolincarboxamid
und pharmazeutisch akzeptablen Salzen davon.

12. Verfahren zur Herstellung einer Verbindung der Formel (I) und pharmazeutisch annehmbaren Salzen davon, wie definiert in einem der Ansprüche 1 bis 11, welches umfaßt:
(A) Umsetzen einer Verbindung der Formel (II): 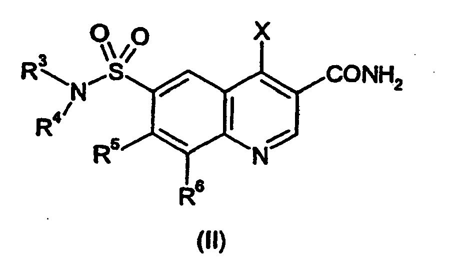 worin R³, R⁴, R⁵ und R⁶ wie oben definiert sind und X ein Halogenatom repräsentiert, mit einem Amin der Formel R¹R²NH, worin R¹ und R² wie oben definiert sind; oder
(B) Interkonversion einer Verbindung der Formel (I) in eine andere Verbindung der Formel (I); oder
(C) Entfernung der Schutzgruppen eines geschützten Derivats einer Verbindung der Formel (I).

13. Verbindung oder pharmazeutische annehmbares Salz davon gemäß einem der Ansprüche 1 bis 11 zur Verwendung in der Therapie.

14. Verwendung einer Verbindung gemäß einem der Ansprüche 1 bis 11, oder eines pharmazeutisch annehmbaren Salzes davon, bei der Herstellung eines Medikaments für die Behandlung oder Prophylaxe entzündlicher und/oder allergischer Erkrankungen.

15. Pharmazeutische Zusammensetzung, die eine Verbindung gemäß einem der Ansprüche 1 bis 11 umfaßt oder ein pharmazeutisch annehmbares Salz davon, optional mit einem pharmazeutisch annehmbaren Träger oder Exzipient.

16. Pharmazeutische Zusammensetzung gemäß Anspruch 15, die für eine Inhalationsverabreichung geeignet ist.

17. Pharmazeutische Zusammensetzung gemäß Anspruch 15, die für eine orale Verabreichung geeignet ist.

18. Pharmazeutische Zusammensetzung gemäß Anspruch 15, die für eine topische Verabreichung geeignet ist.

## Revendications

1. Composé de formule (I) ou sel pharmaceutiquement acceptable de celui-ci : 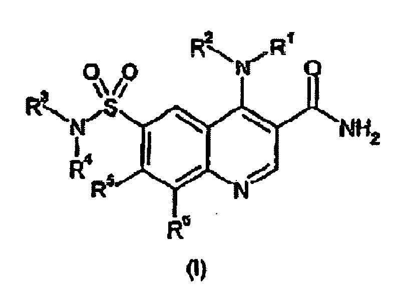 dans lequel :
R¹ est
un groupe aryle facultativement substitué par un ou plusieurs substituants choisis parmi un groupe alkyle en C_{1 à 6}, alcoxy en C_{1 à 6}, un atome d'halogène, un groupe alkyl en C_{1 à 6}CO- , -(CH₂)ₘOH,- CN, R⁷R⁸N- *;*
un groupe aryle condensé à un cycle cycloalkyle en C_{4 à 7} ;
un groupe aryle condensé à un cycle hétérocyclyle;
un groupe hétéroaryle dans lequel le groupe hétéroaryle est facultativement substitué par un ou plusieurs substituants choisis parmi : un groupe alkyle en C_{1 à 6}, N-oxyde, alcoxy en C_{1 à 6} ;
un groupe hétérocyclyle,
R² est un atome d'hydrogène ou un groupe alkyle en C_{1 à 6} ;
R³ est
un atome d'hydrogène ;
un groupe alkyle en C_{1 à 6} facultativement substitué par un ou plusieurs substituants choisis parmi : un groupe hétérocyclyle (lui-même facultativement substitué par un groupe alkyle en C_{1 à 6}) , R⁹ R¹⁰NCO-, R¹¹CONR¹²-, alkyle en C_{1 à 6}SO₂NR¹³-, un groupe alcoxy en C_{1 à 6}, , R¹⁴ R¹⁵ N- ;
un groupe cycloalkyle en C_{3 à 7}
un groupe aryle ou aryl(alkyle en C_{1 à 6}) dans lequel l'aryle est facultativement substitué par un ou plusieurs substituants choisis parmi : un groupe alkyle en C₁ à ₆, alcoxy en C_{1 à 6}, un atome d'halogène, R¹⁶R¹⁷NCO-
un groupe aryle condensé à un groupe cycloalkyle en C_{4 à 7}, dans lequel le cycloalkyle est facultativement substitué par =O ;
un groupe hétéroaryle ou hétéroaryl(alkyle en C_{1 à 6}), dans lequel l'hétéroaryle est facultativement substitué par un ou plusieurs substituants choisis parmi un groupe alkyle en C_{1 à 6}, alcoxy en C₁ à ₆, un atome d'halogène ;
un groupe hétérocyclyle facultativement substitué par un ou plusieurs groupes alkyle en C_{1 à 6}, alkyl en C₁ à ₆CO, alkyl en C_{1 à 6}SO₂-, R¹⁸R¹⁹NCO-, alcoxy en C_{1 à 6}CO- ;
R⁴ est un atome d'hydrogène ou un groupe alkyle en C_{1 à 6} ;
R³ et R⁴ conjointement avec l'atome d'azote auquel ils sont attachés peuvent former un cycle hétérocyclyle, qui est facultativement substitué par un ou plusieurs substituants choisis parmi un groupe alkyle en C_{1 à 6} (facultativement substitué par un ou plusieurs groupes OH ou alcoxy en C_{1 à 6}), alcoxy en C_{1 à 6}, alcoxy en C₁ à ₆CO-, cycloalkyle en C_{3 à 7} (facultativement substitué par OH), alkyl en C_{1 à 6}CO-, alkyl en C_{1 à 6}SO₂-, OH, - (CH₂)ₘNR²⁰R²¹, - (CH₂)ₘCONR²²R²³, - (CH₂)ₘNR²⁴COR²⁵, alcoxy en C_{1 à 6}-alkyle en C_{1 à 4}, arylCO-hétéroaryle, hétéroaryl-alkyle en C_{1 à 4}, hétéroarylCO,
m vaut 0 à 6
R⁵ est un atome d'hydrogène ou un groupe alkyle en C_{1 à 6 ;}
R⁶ est un atome d'hydrogène, un groupe alkyle en C₁ à ₆, alcoxy en C_{1 à 6}, un atome de fluor, de chlore ou de brome ;
R^{7 à 25} représentent tous indépendamment un atome d'hydrogène, un groupe alkyle en C_{1 à 6} ;
R¹⁴ et R¹⁵ conjointement avec l'atome d'azote auquel ils sont attachés peuvent former un cycle hétérocyclique ;
R¹⁶ et R¹⁷ conjointement avec l'atome d'azote auquel ils sont attachés peuvent former un cycle hétérocyclique ;
R¹⁸ et R¹⁹ conjointement avec l'atome d'azote auquel ils sont attachés peuvent former un cycle hétérocyclique ;
R²⁰ et R²¹ conjointement avec l'atome d'azote auquel ils sont attachés peuvent former un cycle hétérocyclique ;
R²² et R²³ conjointement avec l'atome d'azote auquel ils sont attachés peuvent former un cycle hétérocyclique,
dans lequel
- le groupe aryle est un système de cycle aromatique carbocyclique mono- ou bi-cyclique contenant jusqu'à 10 atomes de carbone dans le système de cycle
- le groupe hétéroaryle est un cycle aromatique hétérocyclique monocyclique à cinq à sept chaînons contenant un ou plusieurs hétéroatomes choisis parmi l'oxygène, l'azote et le soufre ou un système de cycles aromatiques hétérocycliques bicycliques condensés contenant au moins un hétéroatome choisi parmi l'oxygène, l'azote et le soufre
- le groupe hétérocyclyle est un cycle monocyclique saturé ou non aromatique, insaturé de trois à sept chaînons contenant au moins un hétéroatome choisi parmi l'oxygène, l'azote et le soufre.

2. Composé selon la revendication 1, dans lequel
R¹ est choisi parmi
un groupe aryle facultativement substitué par un ou plusieurs substituants choisis parmi un groupe alkyle en C_{1 à 6}, alcoxy en C_{1 à} 6, un atome d'halogène, CN ;
un groupe aryle condensé à un cycle hétérocyclyle ;
un groupe hétéroaryle facultativement substitué par un ou plusieurs substituants choisis parmi : un groupe alkyle en C_{1 à 6}.

3. Composé selon la revendication 1 ou 2, dans lequel R² est un atome d'hydrogène.

4. Composé selon l'une quelconque des revendications 1 à 3, dans lequel R³ est choisi parmi
un groupe alkyle en C_{1 à 6} facultativement substitué par un ou plusieurs substituants choisis parmi un groupe hétérocyclyle, alcoxy en C_{1 à 6} ;
un groupe cycloalkyle en C_{3 à 7}
un groupe hétérocyclyle.

5. Composé selon l'une quelconque des revendications 1 à 4, dans lequel R⁴ est un atome d'hydrogène ou un groupe alkyle en C_{1 à 6}.

6. Composé selon l'une quelconque des revendications 1 à 3, dans lequel R³ et R⁴ conjointement avec l'atome d'azote auquel ils sont attachés forment un cycle hétérocyclyle, facultativement substitué par un ou plusieurs substituants choisis parmi un groupe alkyle en C_{1 à 6} (facultativement substitué par un ou plusieurs groupes alcoxy en C_{1 à 6}), alkyl en C_{1 à 6}CO, alkyl en C_{1 à 6}SO₂, - (CH₂) ₘCONR²²R²³, - (CH₂) ₘNR²⁰R²¹, hétéroaryle.

7. Composé selon l'une quelconque des revendications 1 à 6, dans lequel R⁵ est un atome d'hydrogène.

8. Composé selon l'une quelconque des revendications 1 à 7, dans lequel R6 est un atome d'hydrogène ou un groupe alkyle en C_{1 à 6}.

9. Composé selon la revendication 1, dans lequel
R¹ est choisi parmi
un groupe phényle facultativement substitué par un ou plusieurs substituants choisis parmi un groupe méthyle, méthoxy, fluoro, chloro, cyano ;
un groupe dihydrobenzofuranyle ;
un groupe indazolyle ou benzimidazolyle facultativement substitué par un groupe méthyle ;
R² est un atome d'hydrogène ;
R³ est choisi parmi
un groupe alkyle en C_{1 à 3} facultativement substitué par un groupe alcoxy en C_{1 à 2} ou un cycle saturé à 5 à 7 chaînons contenant un ou deux hétéroatomes choisis parmi l'azote ou l'oxygène ;
un groupe cycloalkyle en C_{3 à 5} ;
un cycle saturé à 5 à 7 chaînons contenant un hétéroatome qui est l'oxygène ;
R⁴ est un atome d'hydrogène ou un groupe alkyle en C₁ à 6 ;
R⁵ est un atome d'hydrogène ;
R⁶ est un atome d'hydrogène ou un groupe alkyle en C_{1 à} 6.

10. Composé selon la revendication 1, dans lequel
R¹ est choisi parmi
un groupe phényle facultativement substitué par un ou plusieurs substituants choisis parmi un groupe méthyle, méthoxy, fluoro, chloro, cyano ;
un groupe dihydrobenzofuranyle ;
un groupe indazolyle ou benzimidazolyle facultativement substitué par un groupe méthyle ;
R² est un atome d'hydrogène ;
R³ et R⁴ conjointement avec l'atome d'azote auquel ils sont attachés peuvent former un cycle hétérocyclyle à 5 ou 6 chaînons, facultativement substitué par un ou plusieurs substituants choisis parmi un groupe alkyle en C_{1 à 3} (facultativement substitués par un ou plusieurs groupes alcoxy en C_{1 à 2}), alkyl en C_{1 à 3}CO, alkyl en C_{1 à 3}SO₂, -CON(CH₃)₂, -N(CH₃)₂, pyrazinyle, pyridinyle ;
R⁵ est un atome d'hydrogène ;
R⁶ est un atome d'hydrogène ou un groupe alkyle en C_{1 à 6}

11. Composé de formule (I) choisi dans le groupe consistant en
le 6-[(diméthylamino)sulfonyl]-4-{[3-(méthyloxy)phényl]amino)-3-quinoléinecarboxamide ;
le 4-(2,3-dihydro-1-benzofuran-4-ylamino)-6-(4-morpholinylsulfonyl)-3-quinoléinecarboxamide ;
le 6-[(4-acétyl-1-pipérazinyl)sulfonyl]-4-{[4-fluoro-3-(méthyloxy)phényl]amino)-3-quinoléinecarboxamide ;
le 4-{[4-fluoro-3-(méthyloxy)phényl]amino}-6-{[4-(méthylsulfonyl)-1-pipérazinyl]sulfonyl)-3-quinoléinecarboxamide ;
le 6-[(4-acétyl-1-pipérazinyl)sulfonyl]-4-(2,3-dihydro-1-benzofuran-4-ylamino)-3-quinoléinecarboxamide ;
le 4-(2,3-dihydro-1-benzofuran-4-ylamino)-6-{[4-(méthylsulfonyl)-1-pipérazinyl]sulfonyl}-3-quinoléinecarboxamide ;
le 4-(2,3-dihydro-1-benzofuran-4-ylamino)-6-[(diméthylamino)sulfonyl]-3-quinoléinecarboxamide ;
le 6-({4-[(diméthylamino)carbonyl]-1-pipérazinyl)sulfonyl)-4-{[4-fluoro-3-(méthyloxy)phényl]amino}-3-quinoléinecarboxamide ;
le 4-(2,3-dihydro-1-benzofuran-4-ylamino)-6-{[4-(2-pyrazinyl)-1-pipérazinyl]sulfonyl}-3-quinoléinecarboxamide ;
le 4-(2,3-dihydro-1-benzofuran-4-ylamino)-6-({4-[(diméthylamino)carbonyl]-1-pipérazinyl}sulfonyl)-3-quinoléinecarboxamide ;
le 4-(2,3-dihydro-1-benzofuran-4-ylamino)-6-[(tétrahydro-2H-pyran-4-ylamino)sulfonyl]-3-quinoléinecarboxamide ;
le 4-{[4-fluoro-3-(méthyloxy)phényl]amino}-8-méthyl-6-(4-morpholinylsulfonyl)-3-quinoléinecarboxamide
le 4-(2,3-dihydro-1-benzofuran-4-ylamino)-8-méthyl-6-(4-morpholinylsulfonyl)-3-quinoléinecarboxamide
le 8-méthyl-4-[(3-méthylphényl)amino]-6-(4-morpholinylsulfonyl)-3-quinoléinecarboxamide
le 4[(3-fluorophényl)amino]-8-méthyl-6-(4-morpholinylsulfonyl)3-quinoléinecarboxamide
le 4-[(3-cyanophényl)amino]-8-méthyl-6-(4-morpholinylsulfonyl)3-quinoléinecarboxamide
le 4-(2,3-dihydro-1-benzofuran-4-ylamino)-6-{[4-(diméthylamino)-1-pipéridinyl]sulfonyl}-3-quinoléinecarboxamide
le 4-[(3-chlorophényl)amino)-8-méthyl-6-(4-morpholinylsulfonyl)-3-quinoléinecarboxamide
le 8-méthyl-4-[(1-méthyl-1H-indazol-6-yl)amino]-6-(4-morpholinylsulfonyl)-3-quinoléinecarboxamide
le 6-[(4-acétyl)-1-pipérazinyl)sulfonyl]-8-méthyl-4-[(3-méthylphényl)amino]-3-quinoléinecarboxamide
le 6-[(4-acétyl-1-pipérazinyl)sulfonyl]-4-{[4-fluoro-3-(méthoxy)phényl]amino)-8-méthyl-3-quinoléinecarboxamide
le 6-[(4-acétyl-1-pipérazinyl)sulfonyl]-4-(2,3-dihydro-1-benzofuran-4-ylamino)-8-méthyl-3-quinoléinecarboxamide
et les sels pharmaceutiquement acceptables de ceux-ci.

12. Procédé de préparation d'un composé de formule (I) et des sels pharmaceutiquement acceptables de celui-ci tels que définis dans l'une quelconque des revendications 1 à 11, qui comprend :
(A) faire réagir un composé de formule (II) ; 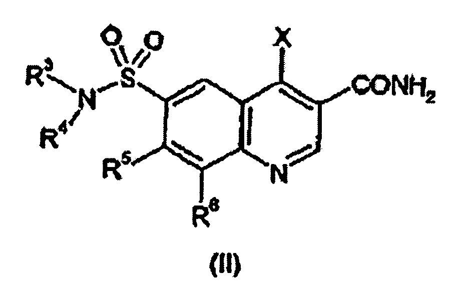 dans laquelle R³ R⁴, R⁵ et R⁶ sont tels que définis ci-dessus, et X représente un atome d'halogène, avec une amine de formule R¹R²NH, dans laquelle R¹ et R² sont tels que définis ci-dessus ; ou
(B) interconvertir un composé de formule (I) en un autre composé de formule (I) ; ou
(C) déprotéger un dérivé protégé d'un composé de formule (I).

13. Composé ou sel pharmaceutiquement acceptable de celui-ci selon l'une quelconque des revendications 1 à 11, à utiliser en thérapie.

14. Utilisation d'un composé selon l'une quelconque des revendications 1 à 11, ou d'un sel pharmaceutiquement acceptable de celui-ci, dans la fabrication d'un médicament destiné au traitement ou à la prophylaxie de maladies inflammatoires et/ou allergiques.

15. Composition pharmaceutique qui comprend un composé selon l'une quelconque des revendications 1 à 11, ou un sel pharmaceutiquement acceptable de celui-ci, facultativement avec un support ou excipient pharmaceutiquement acceptable.

16. Composition pharmaceutique selon la revendication 15, qui est appropriée pour une administration inhalée.

17. Composition pharmaceutique selon la revendication 15, qui est appropriée pour une administration orale.

18. Composition pharmaceutique selon la revendication 15, qui est appropriée pour une administration topique.
